# EUROPEAN PATENT SPECIFICATION

(11) **EP 3 856 130 B1**
(45) Date of publication and mention of the grant of the patent: **12.06.2024**
(21) Application number: 20702577.6
(22) Date of filing: 16.01.2020
(51) Int. Cl.: A61K 8/31, A61K 8/34, A61K 8/35, A61K 8/36, A61Q 13/00, A61Q 15/00, A61K 8/92, C11B 9/00

(54) **ANTIPERSPIRANT OR DEODORANT COMPOSITION**
ANTIPERSPIRANT ODER DEODORIERENDE ZUSAMMENSETZUNG
COMPOSITION ANTIPERSPIRANTE OU DÉODORANTE

(30) Priority: 17.01.2019 EP 19152215
(43) Date of publication of application: 04.08.2021
(73) Proprietor: Firmenich SA, 1242 Satigny (CH)
(72) Inventor: THERRIEN, Mylene, 1242 Satigny (CH); HURRY, Simon, Middlesex UB2 5NN (GB); SUMMERS, Jane, Middlesex UB2 5NN (GB); STRUILLOU, Arnaud, 1242 Satigny (CH)
(74) Representative: Strych, Sebastian
(86) International application number: PCT/EP2020/051037
(87) International publication number: WO 2020/148392

(56) References cited:
- EP-A1- 2 204 155
- WO-A1-00/76472
- WO-A1-2014/029695
- WO-A1-2016/135193
- US-A1- 2014 170 101

## Description

### Technical Field

The present disclosure relates to the field of perfumery. In particular, the present disclosure provides a deodorant or an antiperspirant composition comprising perfume raw materials defined by LogP and used according specific proportions. A dispensing device comprising the composition is also an object of the present invention.

### Background of the Invention

One of the problems faced by the perfumery industry lies in the relatively rapid loss of the olfactive benefit provided by odoriferous compounds due to their volatility. The perfume industry has a particular interest for compositions or additives which are capable of prolonging or enhancing the perfuming effect of a mixture of several fragrances at the same time over a certain period of time. It is particularly desirable to obtain long-lasting properties for standard perfumery raw materials which are too volatile or have a poor substantivity by themselves, or which are only deposited in a small amount onto the surface of the final application.

Furthermore, fragrances play an important role in the perception of products performance and thus they often determine the consumer's choice for a given product. In detergents, hard surface cleaners or personal- or body-care products, the fragrances are incorporated as a free oil and/or encapsulated in microcapsules in order to deliver a pleasant odor to the skin or to the fabrics.

It would be interesting to have an antiperspirant or deodorant composition providing a dual effect with a change of olfactive character over time while showing a boost of freshness upon application.

The present disclosure provides a solution with an antiperspirant or deodorant composition comprising perfume raw materials defined by LogP and used according specific proportions.

### Summary of the Invention

A first object of the invention is therefore an antiperspirant or deodorant composition comprising:
- an antiperspirant or deodorant active material;
- optionally a carrier; and
- a perfume composition comprising perfume raw materials;

characterized in that the perfume raw materials comprise:
   - from 0.01% to 50% by weight of a first group A of perfume raw materials having a LogP ≤ 2.5, and
   - from 0.01% to 50% by weight of a second group B of perfume raw materials having a LogP ≥ 4
wherein the sum of perfume raw materials of group A and perfume raw materials of group B is greater than 35% by weight based on the total weight of the perfume raw materials.

A second object of the invention is a perfume composition comprising perfume raw materials; wherein the perfume raw materials comprise:
- from 0.01% to 50% by weight of a first group A of perfume raw materials having a LogP ≤ 2.5, and
- from 0.01% to 50% by weight of a second group B of perfume raw materials having a LogP ≥ 4
wherein the sum of perfume raw materials of group A and perfume raw materials of group B is greater than 35% by weight based on the total weight of the perfume raw materials.

A third object of the invention is the use of a composition as defined above to modify the olfactive character of said composition under wet conditions.

A fourth object of the invention is a method for modifying the olfactive character of a composition comprising the steps consisting of:
- providing a perfume composition as defined above; and
- subjecting said composition to wet conditions, for example sweat.

### Detailed description of of the Invention

The present invention provides an antiperspirant or a deodorant composition comprising a perfume composition including a first group of perfuming compounds formed of raw materials and a second group of perfuming compounds formed of raw materials respectively defined by their LogP values.

The Applicant shows that the olfactive character of the perfume composition as defined in the present invention changes under wet conditions.

Indeed, the use of a perfume composition comprising a specific combination of raw materials defined according to their physico-chemical properties enables to create distinct smells within a single fragrance when said composition is subjected to wet conditions.

In other words, two different fragrance directions can be perceived by a consumer from a single fragrance during the transition from dry stage to wet stage. For example, when sweating, the consumer will perceive a change in freshness and strength perception.

A first object of the invention is therefore an antiperspirant or deodorant composition comprising:
- an antiperspirant or deodorant active material;
- optionally a carrier; and
- a perfume composition comprising perfume raw materials;
characterized in that the perfume raw materials comprise:
- from 0.01% to 50% by weight of a first group A of perfume raw materials having a LogP ≤ 2.5, and
- from 0.01% to 50% by weight of a second group B of perfume raw materials having a LogP ≥ 4
wherein the sum of perfume raw materials of group A and perfume raw materials of group B is greater than 35% by weight based on the total weight of the perfume raw materials.

As used herein, the term "perfume raw materials", refers to a compound or mixture of perfuming ingredients, which are used in a perfuming preparation or composition to impart a hedonic effect. In other words such perfuming ingredients, to be considered as being a perfuming one, must be recognized by a person skilled in the art as being able to impart or modify in a positive or pleasant way the odour of a composition, and not just as having an odour.

As used herein, the term "perfuming ingredient" it is meant a compound, which is used for the primary purpose of conferring or modulating an odour. In other words such an ingredient, to be considered as being a perfuming one, must be recognized by a person skilled in the art as being able to at least impart or modify in a positive or pleasant way the odour of a composition, and not just as having an odour. For the purpose of the present disclosure, perfume accord also includes combination of perfuming ingredients with substances which together improve, enhance or modify the delivery of the perfuming ingredients, such as perfume precursors, emulsions or dispersions, as well as combinations which impart an additional benefit beyond that of modifying or imparting an odour, such as long-lasting, blooming, malodour counteraction, antimicrobial effect, microbial stability, insect control.

The nature and type of the perfuming ingredients do not warrant a more detailed description here, which in any case would not be exhaustive, the skilled person being able to select them on the basis of its general knowledge and according to intended use or application and the desired organoleptic effect. In general terms, these perfuming ingredients belong to chemical classes as varied as alcohols, aldehydes, ketones, esters, ethers, acetates, nitriles, terpenoids, nitrogenous or sulphurous heterocyclic compounds and essential oils, and said perfuming co-ingredients can be of natural or synthetic origin. Many of these ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said ingredients may also be compounds known to release in a controlled manner various types of perfuming compounds.

According to the invention, the perfume composition can comprise in addition to perfume raw materials, at least one solvent to dissolve said perfume raw materials, which is of current use in the perfumery industry. The solvent is preferably chosen in the group consisting of dipropylene glycol (4-oxa-2,6-heptanediol + 2-methyl-3-oxa-1,5-hexanediol + 2,4-dimethyl-3-oxa-1,5-pentanediol), Isopar M (hydrocarbons C13-C14), Isopar L (hydrocarbons C11 - C13), isopropyl myristate (isopropyl tetradecanoate) ethyle citrate (triethyl 2-hydroxy-1,2,3-propanetricarboxylate), triacetine (1,2,3-propanetriyl triacetate) , 1,3-propanediol, mixture of methyl dihydroabietate and methyl tetrahydroabietate, vegetable oils such as almond oil, argan oil, cotton oil, corn oil, olive oil, sunflower oil, castor oil and mixtures thereof.

In other words, it means that solvents are not included in the perfume raw materials as defined in the present invention.

According to the invention, the perfume raw materials comprise:
- from 0.01% to 50% by weight of a first group A of perfume raw materials having a LogP ≤ 2.5, and
- from 0.01% to 50% by weight of a second group B of perfume raw materials having a LogP ≥ 4
wherein the sum of perfume raw materials of group A and perfume raw materials of group B is greater than 35% by weight based on the total weight of the perfume raw materials.

According to an embodiment, the sum of perfume raw materials of group A and perfume raw materials of group B is comprised between 35% and 80% by weight based on the total weight of the perfume raw materials.

The skilled person will be able to select the raw materials from the first group and raw materials of the second group according to their LogP value on the basis of his general knowledge. LogP is the common logarithm of estimated octanol-water partition coefficient, which is known as a measure of lipophilicity.

The LogP values of many perfuming compound have been reported, for example, in the Pomona92 database, available from Daylight Chemical Information Systems, Inc. (Daylight CIS), Irvine, Calif., which also contains citations to the original literature. LogP values are most conveniently calculated by the "CLOGP" program, also available from Daylight CIS. This program also lists experimental logP values when they are available in the Pomona92 database. The "calculated logP" (cLogP) is determined by the fragment approach of Hansch and Leo (cf., A. Leo, in Comprehensive Medicinal Chemistry, Vol. 4, C. Hansch, P. G. Sammens, J. B. Taylor and C. A. Ramsden, Eds., p. 295, Pergamon Press, 1990). The fragment approach is based on the chemical structure of each perfume oil ingredient, and takes into account the numbers and types of atoms, the atom connectivity, and chemical bonding. The cLogP values, which are the most reliable and widely used estimates for this physicochemical property, are preferably used instead of the experimental LogP values in the selection of perfuming compounds which are useful in the present invention.

Thus, by adjusting a correct balance between raw materials having a low LogP and raw materials having a high LogP, a "2 in 1" fragrance is obtained where two different olfactive directions are provided under wet conditions in conjunction with an increase in intensity (boost).

According to an embodiment, the first group of perfuming compounds is formed of perfume raw material having a LogP ≤ 2.5 and the second group of perfuming compounds is made of perfume raw materials having a LogP ≥ 4.5.

According to another embodiment, the perfume raw materials comprise between 2-25% by weight of the first group A of perfume raw materials and between 2-25% by weight of the second group B of perfume raw materials.

According to a particular embodiment, the sum of perfume raw materials of group A and perfume raw materials of group B having an odor detection threshold (ODT) ≤ 2 × 10⁻³ µg/L is greater than 8 %, preferably between 8 and 80%, more preferably between 15 and 80% based on the total weight of the perfume raw materials.

According to the invention, the term "Odour Detection Threshold" refers to the lowest vapour concentration of that material which can be olfactorily detected.

The odor threshold concentration of a perfuming compound is determined by using a gas chromatograph ("GC"). Specifically, the gas chromatograph is calibrated to determine the exact volume of the perfume oil ingredient injected by the syringe, the precise split ratio, and the hydrocarbon response using a hydrocarbon standard of known concentration and chain-length distribution. The air flow rate is accurately measured and, assuming the duration of a human inhalation to last 12 seconds, the sampled volume is calculated. Since the precise concentration at the detector at any point in time is known, the mass per volume inhaled is known and hence the concentration of the perfuming compound. To determine the threshold concentration, solutions are delivered to the sniff port at the back-calculated concentration. A panelist sniffs the GC effluent and identifies the retention time when odor is noticed. The average across all panelists determines the odor threshold concentration of the perfuming compound. The determination of odor threshold is described in more detail in C. Vuilleumier et al., Multidimensional Visualization of Physical and Perceptual Data Leading to a Creative Approach in Fragrance Development, Perfume & Flavorist, Vol. 33, September" 2008, pages 54-61.

According to a particular embodiment, the perfume composition further comprises at least 10% by weight of perfume raw materials of group C having a log P comprised between 2.5 and 4 (2.5 and 4 excluded) and/or an ODT with an odor detection threshold (ODT) of ≤ 2 × 10⁻³ µg/L.

As non-limiting examples of perfume raw materials with logP≤2.5 and ODT≤2 × 10⁻³ ug/L air, one may cite compounds listed in table A below.

**Table A: Perfume raw materials with logP≤2.5 and ODT≤2 × 10⁻³ ug/L**

| **CHEMICAL NAME** |
|---|
| 2-(4-METHYL-1,3-THIAZOL-5-YL)-1-ETHANOL |
| 3-ETHOXY-4-HYDROXYBENZALDEHYDE |
| 2-ETHYL-3-HYDROXY-4(4H)-PYRANONE |
| 4-(4-HYDROXYPHENYL)-2-BUTANONE |
| 7-HYDROXY-2-CHROMENONE |
| PHENYL ETHYL ALCOHOL |
| ALDEHYDE ANISIQUE |
| CINNAMIC ALCOHOL |
| 4-NONANOLIDE |
| 2-METHOXY-4-(2-PROPEN-1-YL)PHENOL |
| METHYL 2,4-DIHYDROXY-3,6-DIMETHYLBENZOATE |
| PHENYL ETHYL ACETATE |
| VANILLIN |
| ISOEUGENOL |
| (3ARS,6SR,7ASR)-PERHYDRO-3,6-DIMETHYL-BENZO[B]FURAN-2-ONE |
| ETHYL BUTYRATE |
| METHYL NAPHTYL KETONE |
| 7-METHYL-2H-1,5-BENZODIOXEPIN-3(4H)-ONE |
| BENZYL ACETONE |
| 3-(1,3-BENZODIOXOL-5-YL)-2-METHYLPROPANAL |
| 2,4-DIMETHYL-3-CYCLOHEXENE-1-CARBALDEHYDE |
| (+-)-3-PHENYLBUTANAL |
| BENZYLACETONE |
| 4-(1,3-BENZODIOXOL-5-YL)-2-BUTANONE |
| (+-)-5-ETHYL-4-HYDROXY-2-METHYL-3(2H)-FURANONE (A) + (+-)-2-ETHYL-4-HYDROXY-5-METHYL-3(2H)-FURANONE (B) |
| ETHYL ISOBUTYRATE |
| ETHYL METHYLPHENYLGLYCIDATE |
| ETHYL 2-METHYL-1,3-DIOXOLANE-2-ACETATE |
| 1-PHENYLVINYL ACETATE |

As non-limiting examples of perfume raw materials with logP≥4 and ODT≤2 × 10⁻³ ug/L air, one may cite perfume raw materials of table B.

**Table B: Perfume raw materials with logP≥4 and ODT≤2 × 10⁻³ ug/L air**

| **CHEMICAL NAME** |
|---|
| (4Z)-4-DODECENAL |
| 3,3-DIMETHYL-5-(2,2,3-TRIMETHYL-3-CYCLOPENTEN-1-YL)-4-PENTEN-2-OL |
| {1-METHYL-2-[(1,2,2-TRIMETHYLBICYCLO[3.1.0]HEX-3-YL)METHYL]CYCLOPROPYL}METHANOL |
| 2-METHYLUNDECANAL |
| (1S,1'R)-2-[1-(3',3'-DIMETHYL-1'-CYCLOHEXYL)ETHOXY]-2-METHYLPROPYLPROPANOATE |
| (3AR,5AS,9AS,9BR)-3A,6,6,9A-TETRAMETHYLDODECAHYDRONAPHTHO[2,1-B]FURAN |
| 1-(2,2,3,6-TETRAMETHYL-CYCLOHEXYL)-3-HEXANOL |
| 1-(OCTAHYDRO-2,3,8,8-TETRAMETHYL-2-NAPHTALENYL)-1-ETHANONE |
| (2E)-TRIDEC-2-ENITRILE |
| VERDYL PROPIONATE |
| NAPHTHO[2,1-B]FURAN, DODECAHYDRO3A,6,6,9A-TETRAMETHYL |
| PATCHOULOL |
| ALDEHYDE C12 |
| 1-[(1RS,6SR)-2,2,6-TRIMETHYLCYCLOHEXYL]-3-HEXANOL |
| (+)-(3R)-1-[(1R,6S)-2,2,6-TRIMETHYLCYCLOHEXYL]-3-HEXANOL |
| (3E,5Z)-1,3,5-UNDECATRIENE |
| PENTADECENOLIDE |
| (+-)-2-(4-METHYL-3-CYCLOHEXEN-1-YL)-2-PROPANETHIOL |
| ALDEYHYDE SUPRA |
| ((-)-(2E)-2-ETHYL-4-[(1R)-2,2,3-TRIMETHYL-3-CYCLOPENTEN-1-YL]-2-BUTEN-1-OL |
| 4-PENTEN-1-OL, 2-METHYL-4-(2,2,3-TRIMETHYL-3-CYCLOPENTEN-1-YL)- |
| IONONE, METHYL |
| 3-MÉTHYL-CYCLOPENTADÉCANONE |
| 1-(2,3,8,8-TETRAMETHYL-1,3,4,5,6,7-HEXAHYDRONAPHTHALEN-2-YL)ETHANONE |
| ALDEHYDE HEXYLCINNAMIQUE |
| (+-)-(1S,4AR,8S,8AR)-2,2,6,8-TETRAMETHYL-1,2,3,4,4A,5,8,8A-OCTAHYDRO-1-NAPHTHALENOL |
| OXACYCLOHEXADECAN-2-ONE |
| MUSCENONE DELTA |
| DELTA DAMASCONE |

As non-limiting examples of perfume raw materials having a log P comprised between 2.5 and 4 and an ODT with the odor detection threshold (ODT) of ≤ 2 × 10⁻³ µg/L, one may cite linalyl Acetate, benzyl benzoate, , dihydromyrcenol, , linalol, sclareolate ((-)-propyl (S)-2-(1,1-dimethylpropoxy)propanoate), ethyl acetoacetate, and mixtures thereof.

According to an embodiment, the perfume composition comprises at least one solvent to solubilized the perfume raw materials, said solvent is preferably chosen in the group consisting of dipropylene glycol, Isopar M (hydrocarbons C₁₃-C₁₄), Isopar L (hydrocarbons C₁₁ - C₁₃), isopropyl myristate (isopropyl tetradecanoate) ethyle citrate (triethyl 2-hydroxy-1,2,3-propanetricarboxylate), triacetine (1,2,3-propanetriyl triacetate) , benzyl benzoate , 1,3-propanediol, mixture of methyl dihydroabietate and methyl tetrahydroabietate, vegetable oils such as almond oil, argan oil, cotton oil, corn oil, olive oil, sunflower oil, castor oil and mixtures thereof.

When present, the solvent may be comprised up to 50%, preferably up to 30% by weight of the perfume composition.

The perfume composition of the invention can be used as a free oil and/or in an encapsulated form.

The encapsulated form can be microcapsules which have been widely described in the prior art. One may cite for example the core-shell type with a polymeric shell or microcapsules having a polymeric matrix made of a water soluble polymer, for example a starch-based water soluble polymer. Microcapsules having a polymeric matrix can be obtained by spray-drying.

The nature of the polymeric shell from the microcapsules of the invention can vary. As non-limiting examples, the shell can be aminoplast-based, polyurea-based or polyurethane-based. The shell can also be hybrid, namely organic-inorganic such as a hybrid shell composed of at least two types of inorganic particles that are cross-linked, or yet a shell resulting from the hydrolysis and condensation reaction of a polyalkoxysilane macro-monomeric composition.

According to an embodiment, the shell comprises an aminoplast copolymer, such as melamine-formaldehyde or urea-formaldehyde or cross-linked melamine formaldehyde or melamine glyoxal.

According to another embodiment the shell is polyurea-based made from, for example but not limited to isocyanate-based monomers and amine-containing crosslinkers such as guanidine carbonate and/or guanazole. Preferred polyurea microcapsules comprise a polyurea wall which is the reaction product of the polymerisation between at least one polyisocyanate comprising at least two isocyanate functional groups and at least one reactant selected from the group consisting of an amine (for example a water soluble guanidine salt and guanidine); a colloidal stabilizer or emulsifier; and an encapsulated perfume. However, the use of an amine can be omitted.

According to a particular embodiment the colloidal stabilizer includes an aqueous solution of between 0.1% and 0.4% of polyvinyl alcohol, between 0.6% and 1% of a cationic copolymer of vinylpyrrolidone and of a quaternized vinylimidazol (all percentages being defined by weight relative to the total weight of the colloidal stabilizer). According to another embodiment, the emulsifier is an anionic or amphiphilic biopolymer preferably chosen from the group consisting of gum Arabic, soy protein, gelatin, sodium caseinate and mixtures thereof.

According to another embodiment, the shell is polyurethane-based made from, for example but not limited to polyisocyanate and polyols, polyamide, polyester, etc.

The preparation of an aqueous dispersion/slurry of core-shell microcapsules is well known by a skilled person in the art. In one aspect, said microcapsule wall material may comprise any suitable resin and especially including melamine, glyoxal, polyurea, polyurethane, polyamide, polyester, etc. Suitable resins include the reaction product of an aldehyde and an amine, suitable aldehydes include, formaldehyde and glyoxal. Suitable amines include melamine, urea, benzoguanamine, glycoluril, and mixtures thereof. Suitable melamines include, methylol melamine, methylated methylol melamine, imino melamine and mixtures thereof. Suitable ureas include, dimethylol urea, methylated dimethylol urea, urea-resorcinol, and mixtures thereof. Suitable materials for making may be obtained from one or more of the following companies Solutia Inc. (St Louis, Missouri U.S.A.), Cytec Industries (West Paterson, New Jersey U.S.A.), Sigma-Aldrich (St. Louis, Missouri U.S.A.).

According to a particular embodiment, the core-shell microcapsule is a formaldehyde-free capsule. A typical process for the preparation of aminoplast formaldehyde-free microcapsules slurry comprises the steps of 1) preparing an oligomeric composition comprising the reaction product of, or obtainable by reacting together
a) a polyamine component in the form of melamine or of a mixture of melamine and at least one C₁-C₄ compound comprising two NH₂ functional groups;
b) an aldehyde component in the form of a mixture of glyoxal, a C₄-₆ 2,2-dialkoxy-ethanal and optionally a glyoxalate, said mixture having a molar ratio glyoxal/C₄-₆ 2,2-dialkoxy-ethanal comprised between 1/1 and10/1; and
c) a protic acid catalyst;
   2) preparing an oil-in-water dispersion, wherein the droplet size is comprised between 1 and 600 um, and comprising:
   i. an oil;
   ii. a water medium
   iii. at least an oligomeric composition as obtained in step 1;
   iv. at least a cross-linker selected amongst

A) C₄-C₁₂ aromatic or aliphatic di- or tri-isocyanates and their biurets, triurets, trimmers, trimethylol propane-adduct and mixtures thereof; and/or
B) a di- or tri-oxiran compounds of formula
   A-(oxiran-2-ylmethyl)ₙ
   wherein n stands for 2 or 3 and 1 represents a C₂-C₆ group optionally comprising from 2 to 6 nitrogen and/or oxygen atoms;
      v. optionally a C₁-C₄ compounds comprising two NH₂ functional groups;
3) Heating said dispersion;
4) Cooling said dispersion.

This process is described in more details in WO 2013/068255.

According to another embodiment, the shell of the microcapsule is polyurea-or polyurethane-based. Examples of processes for the preparation of polyurea and polyureathane-based microcapsule slurry are for instance described in WO2007/004166, EP 2300146, EP2579976. Typically a process for the preparation of polyurea or polyurethane-based microcapsule slurry include the following steps:
a) Dissolving at least one polyisocyanate having at least two isocyanate groups in an oil to form an oil phase;
b) Preparing an aqueous solution of an emulsifier or colloidal stabilizer to form a water phase;
c) Adding the oil phase to the water phase to form an oil-in-water dispersion, wherein the mean droplet size is comprised between 1 and 500 µm, preferably between 5 and 50 µm;
d) Applying conditions sufficient to induce interfacial polymerisation and form microcapsules in form of a slurry.

### Antiperspirant or deodorant products

The compositions presented herein may be incorporated into any antiperspirant or deodorant product. Exemplary products include wax-based sticks, soap-based sticks, compressed powder sticks, roll-on suspensions or solutions, emulsions, gels, creams, squeeze sprays, pump sprays, aerosols, and the like. Each product form may contain its own selection of additional components, some essential and some optional. The types of components typical for each of the above product forms may be incorporated in the corresponding compositions presented herein.

As used herein, the term "antiperspirant or deodorant product" refers to the normal meaning in the art; i.e. a composition applied on skin allowing to reduce or prevent body odour. Suitable deodorant actives can include any topical material that is known or otherwise effective in preventing or eliminating malodour, including malodour associated with sweat and/or perspiration. Suitable deodorant actives may be selected from the group consisting of antimicrobial agents (e.g., bacteriocides, fungicides), malodour-absorbing material, ethylhexylglycerin, alcohol such as ethanol and combinations thereof.

Antiperspirant actives may include astringent metallic salts, especially inorganic and organic salts of aluminum, zirconium and zinc, as well as mixtures thereof. Even more specifically, antiperspirant actives may be selected from the group consisting of aluminum chloride, aluminum chlorohydrate, aluminum chlorohydrex, aluminum chlorohydrex PG, aluminum chlorohydrex PEG, aluminum dichlorohydrate, aluminum dichlorohydrex PG, aluminum dichlorohydrex PEG, aluminum sesquichlorohydrate, aluminum sesquichlorohydrex PG, aluminum sesquichlorohydrex PEG, aluminum sulfate, aluminum zirconium octachlorohydrate, aluminum zirconium octachlorohydrex GLY, aluminum zirconium pentachlorohydrate, aluminum zirconium pentachlorohydrex GLY, aluminum zirconium tetrachlorohydrate, aluminum zirconium trichlorohydrate, aluminum zirconium tetrachlorohydrate GLY and aluminum zirconium trichlorohydrate GLY.

Depending of the type of product, the deodorant or antiperspirant product may comprise supplementary ingredients enabling to obtain the desired form. Non-limiting examples of suitable ingredients include emollient(s), solubilizer(s), antioxidant(s), preservative(s), carrier(s), odour entrapper(s), propellant(s), primary structurant(s), additional chassis ingredient(s), volatile silicone solvent(s), gellant(s), buffering agent and residue masking material(s). A person skilled in the art is able to select them on the basis of its general knowledge and according to intended form of the deodorant or antiperspirant composition.

For example, by way of illustration, a roll-on deodorant or antiperspirant product may comprise water, emollient, solubilizer, deodorant or antiperspirant actives, antioxidants, preservatives, or combinations thereof; a clear gel product or antiperspirant product may comprise water, emollient, solubilizer, deodorant or antiperspirant actives, antioxidants, preservatives, ethanol, or combinations thereof; a body spray may contain a carrier, deodorant or antiperspirant actives, odour entrappers, propellant, or combinations thereof; an invisible solid deodorant or antiperspirant product may contain a primary structurant, deodorant or antiperspirant actives, and additional chassis ingredient(s); a soft solid deodorant or antiperspirant product may comprise volatile silicone, deodorant or antiperspirant actives, gellant, residue masking material, or combinations thereof; an aerosol deodorant or antiperspirant product may comprise a carrier, a propellant, or a combination thereof.

Emollients suitable for deodorant or antiperspirant products include, but are not limited to, propylene glycol, polypropylene glycol (like dipropylene glycol, tripropylene glycol, etc.), diethylene glycol, triethylene glycol, neopentyl glycol diheptanoate, PEG-4, PEG-8, 1,2-pentanediol, 1,2-hexanediol, hexylene glycol, glycerin, C₂ to C₂₀ monohydric alcohols, C₂ to C₄₀ dihydric or polyhydric alcohols, alkyl ethers of polyhydric and monohydric alcohols, dicaprylyl carbonate, dicaprylyl ether, diethylhexylcyclohexane, dibutyl adipate, volatile silicone emollients such as cyclopentasiloxane, nonvolatile silicone emollients such as dimethicone, mineral oils, polydecenes, petrolatum, and combinations thereof. One example of a suitable emollient comprises PPG-15 stearyl ether. Other examples of suitable emollients include dipropylene glycol and propylene glycol.

Antimicrobial agents may comprise cetyl-trimethylammonium bromide, cetyl pyridinium chloride, benzethonium chloride, diisobutyl phenoxy ethoxy ethyl dimethyl benzyl ammonium chloride, sodium N-lauryl sarcosine, sodium N-palmethyl sarcosine, lauroyl sarcosine, N-myristoyl glycine, potassium N-lauryl sarcosine, trimethyl ammonium chloride, sodium aluminum chlorohydroxy lactate, triethyl citrate, tricetylmethyl ammonium chloride, 2,4,4'-trichloro-2'hydroxy diphenyl ether (triclosan), 3,4,4'-trichlorocarbanilide (triclocarban), diaminoalkyl amides such as L-lysine hexadecyl amide, heavy metal salts of citrate, salicylate, and piroctose, especially zinc salts, and acids thereof, heavy metal salts of pyrithione, especially zinc pyrithione, zinc phenolsulfate, farnesol, and combinations thereof.

Suitable odour entrappers for use herein include, for example, solubilized, water-soluble, uncomplexed cyclodextrin. As used herein, the term "cyclodextrin" includes any of the known cyclodextrins such as unsubstituted cyclodextrins containing from six to twelve glucose units, including alpha-cyclodextrin, beta-cyclodextrin, gamma-cyclodextrin and/or their derivatives and/or mixtures thereof.

Alternative malodour entrappers can be zinc ricinoleate & derivatives such as TEGO^{®} SORB B 80, TEGO ^{®} Sorb Conc. 50& TEGO^{®} SORB A 30.

A suitable solubilizer can be, for example, a surfactant, such as a no-foaming or lowfoaming surfactant. Suitable surfactants are nonionic surfactants, cationic surfactants, amphoteric surfactants, zwitterionic surfactants, and mixtures thereof. Suitable solubilizers include, for example, polyethylene glycol ether of cetearyl alcohol, hydrogenated castor oil such as polyoxyethylene hydrogenated castor oil, polyoxyethylene 2 stearyl ether, polyoxyethylene 20 stearyl ether, and combinations thereof.

Suitable preservatives include organic sulfur compounds, halogenated compounds, cyclic organic nitrogen compounds, low molecular weight aldehydes, parabens, propane diol materials, isothiazolinones, quaternary compounds, benzoates, low molecular weight alcohols, dehydroacetic acid, phenyl and phenoxy compounds, or mixtures thereof.

Non-limiting examples of commercially available preservatives include a mixture of about 77% 5-chloro-2-methyl-4-isothiazolin-3-one and about 23% 2-methyl-4-isothiazolin-3-one, a broad spectrum preservative available as a 1.5% aqueous solution under the trade name Kathan^{®} CG by Rohm and Haas Co.; 5-bromo-5-nitro-1,3-dioxane, available under the tradename Bronidox L^{®} from Henkel; 2-bromo-2-nitropropane-1,3-diol, available under the trade name Bronopol^{®} from Inolex; 1,1'-hexamethylene bis(5-(p-chlorophenyl)biguanide), commonly known as chlorhexidine, and its salts, e.g., with acetic and digluconic acids; a 95:5 mixture of 1,3-bis(hydroxymethyl)-5,5-dimethyl-2,4-imidazolidinedione and 3-butyl-2-iodopropynyl carbamate, available under the trade name Glydant Plus^{®} from Lonza; N-[1,3-bis(hydroxymethyl)2,5-dioxo-4-imidazolidinyl]-N,N'-bis(hydroxy-methyl) urea, commonly known as diazolidinyl urea, available under the trade name Germall^{®} II from Sutton Laboratories, Inc.; N,N"-methylenebis {N'-[1-(hydroxymethyl)-2,5-dioxo-4-imidazolidinyl]urea}, commonly known as imidazolidinyl urea, available, e.g., under the trade name Abiol^{®} from 3V-Sigma, Unicide U-13^{®} from Induchem, German 115^{®} from Sutton Laboratories, Inc.; polymethoxy bicyclic oxazolidine, available under the trade name Nuosept^{®} C from Hills America; formaldehyde; glutaraldehyde; polyaminopropyl biguanide, available under the trade name Cosmocil CQ^{®} from ICI Americas, Inc., or under the trade name Mikrokill^{®} from Brooks, Inc; dehydroacetic acid; and benzsiothiazolinone available under the trade name Koralone^{™} B-119 from Rohm and Hass Corporation.

Suitable levels of preservative can range from about 0.0001 % to about 0.5%, alternatively from about 0.0002% to about 0.2%, alternatively from about 0.0003% to about 0.1 %, by weight of the composition.

Suitable carriers can include, water, alcohol, or combinations thereof. Useful alcohols include C₁-C₃ alcohols. In some aspects, the alcohol is ethanol.

Some examples of propellants include compressed air, nitrogen, inert gases, carbon dioxide, and mixtures thereof. Propellants may also include gaseous hydrocarbons like propane, n-butane, isobutene, cyclopropane, and mixtures thereof; e.g. A-46 (a mixture of isobutane, butane and propane), A-31 (isobutane), A-17 (n-butane), A-108 (propane), AP70 (a mixture of propane, isobutane and nbutane), AP40 (a mixture of propane, isobutene and n-butane), AP30 (a mixture of propane, isobutane and n-butane). Some non-limiting examples of propellants include 1,1-difluoroethane, 1,1,1,2,2-pentafluoroethane, 1,1,1,2-tetrafluoroethane, 1,1,1,2,3,3,3-heptafluoropropane, trans-1,3,3,3-tetrafluoroprop-1-ene, dimethyl ether, dichlorodifluoromethane (propellant 12), 1,1-dichloro-1,1,2,2-tetrafluoroethane (propellant 114 ), 1-chloro-1,1-difluoro-2,2-trifluoroethane (propellant 115), 1-chloro-1,1-difluoroethylene (propellant 142B), 1,1-difluoroethane (propellant 152A), monochlorodifluoromethane, and mixtures thereof.

The term "primary structurant" as used herein means any material known or otherwise effective in providing suspending, gelling, viscosifying, solidifying, and/or thickening properties to the composition or which otherwise provide structure to the final product form. These primary structurants include gelling agents, and polymeric or non-polymeric or inorganic thickening or viscosifying agents. Such materials will typically be solids under ambient conditions and include organic solids, crystalline or other gellants, inorganic particulates such as clays or silicas, or combinations thereof. Non-limiting examples of suitable primary structurants include stearyl alcohol and other fatty alcohols; hydrogenated castor wax (e.g., Castorwax MP80, Castor Wax, etc.); hydrocarbon waxes include paraffin wax, beeswax, carnauba, candelilla, spermaceti wax, ozokerite, ceresin, baysberry, synthetic waxes such as Fischer-Tropsch waxes, and microcrystalline wax; polyethylenes with molecular weight of 200 to 1000 daltons; solid triglycerides; behenyl alcohol, or combinations thereof.

Chassis ingredients may be an additional structurant such as stearyl alcohol and other fatty alcohols; hydrogenated castor wax (e.g., Castorwax MP80, Castor Wax, etc.); hydrocarbon waxes include paraffin wax, beeswax, carnauba, candelilla, spermaceti wax, ozokerite, ceresin, baysberry, synthetic waxes such as Fisher-Tropsch waxes, and microcrystalline wax; polyethylenes with molecular weight of 200 to 1000 daltons; and solid triglycerides; behenyl alcohol, or combinations thereof; non-volatile organic fluids such as mineral oil, PPG-14 butyl ether, isopropyl myristate, petrolatum, butyl stearate, cetyl octanoate, butyl myristate, myristyl myristate, C12-15 alkylbenzoate (e.g., Finsolv.TM.), octyldodecanol, isostearyl isostearate, octododecyl benzoate, isostearyl lactate, isostearyl palmitate or isobutyl stearate; clay mineral powders such as talc, mica, sericite, silica, magnesium silicate, synthetic fluorphlogopite, calcium silicate, aluminum silicate, bentonite and montomorillonite; pearl pigments such as alumina, barium sulfate, calcium secondary phosphate, calcium carbonate, titanium oxide, finely divided titanium oxide, zirconium oxide, zinc oxide, hydroxy apatite, iron oxide, iron titrate, ultramarine blue, Prussian blue, chromium oxide, chromium hydroxide, cobalt oxide, cobalt titanate, titanium oxide coated mica; organic powders such as polyester, polyethylene, polystyrene, methyl methacrylate resin, cellulose, 12-nylon, 6-nylon, styrene-acrylic acid copolymers, poly propylene, vinyl chloride polymer, tetrafluoroethylene polymer, boron nitride, fish scale guanine, laked tar color dyes, laked natural color dyes; and combinations thereof.

Volatile silicone solvents suitable for use in the antiperspirant compositions include, but are not limited to, solvent such as Cyclomethicone D-5; GE 7207 and GE 7158 (commercially available from General Electric Co.); Dow Corning 344; Dow Corning 345; Dow Corning 200; and DC1184 (commercially available from Dow Corning Corp.); and SWS-03314 (commercially available from SWS Silicones).

The gellant material may comprise saturated or unsaturated, substituted or unsubstituted, fatty alcohols or mixtures of fatty alcohols having from about 20 to about 60 carbons atoms, alternatively from about 20 to about 40 carbon atoms. In some embodiments, the gallant materials comprise combinations of the fatty alcohols. In some embodimens, the fatty alcohol gellants are may be saturated, unsubstituted monohydric alcohols or combinations thereof, which have a melting point of at less than about 110°C, alternatively from about 60° to about 110°C, alternatively between about 100 °C and 110°C.

Specific examples of fatty alcohol gellants for use in the antiperspirant products that are commercially available include, but are not limited to, Unilin^{®} 425, Unilin^{®} 350, Unilin^{®} 550 and Unilin^{®} 700 (supplied by Petrolite).

A suitable buffering agent may be alkaline, acidic or neutral. The buffer may be used in the composition or product for maintaining the desired pH. Suitable buffering agents include, for example, hydrochloric acid, sodium hydroxide, potassium hydroxide, and combinations thereof.

Non-limiting examples of suitable residue masking materials for use in the antiperspirant products include butyl stearate, diisopropyl adipate, petrolatum, nonvolatile silicones, octyldodecanol, phenyl trimethicone, isopropyl myristate, C₁₂₋₁₅ ethanol benzoates and PPG-14 Butyl Ether.

The deodorant or antiperspirant products disclosed herein may comprise other optional ingredients such as emulsifiers, distributing agents, antimicrobials, pharmaceutical or other topical actives, surfactants, and the like.

The nature, amount and type of ingredients does not warrant a more detailed description here, which in any case would not be exhaustive, the skilled person being able to select them on the basis of its general knowledge and according to intended form.

In some aspects, the composition comprises less than 95 wt% of water, relative to the total weight of the composition. In some aspects, the composition comprises less than 90 wt% of water, relative to the total weight of the composition. In some aspects, the composition comprises less than 85 wt% of water, relative to the total weight of the composition. In some aspects, the composition comprises less than 80 wt% of water, relative to the total weight of the composition. In some aspects, the composition comprises less than 75 wt% of water, relative to the total weight of the composition. In some aspects, the composition comprises less than 70 wt% of water, relative to the total weight of the composition. In some aspects, the composition comprises less than 65 wt% of water, relative to the total weight of the composition. In some aspects, the composition comprises less than 60 wt% of water, relative to the total weight of the composition. In some aspects, the composition comprises less than 55 wt% of water, relative to the total weight of the composition. In some aspects, the composition comprises less than 50 wt%, or less than 40 wt%, or less than 30 wt%, or less than 20 wt%, or less than 10 wt% of water, relative to the total weight of the deodorant or antiperspirant composition. In some aspects, the composition is water-free.

Another object of the invention is a perfume composition comprising perfume raw materials;
wherein the perfume raw materials comprise:
   - from 0.01% to 50% by weight of a first group A of perfume raw materials having a LogP ≤ 2.5, and
   - from 0.01% to 50% by weight of a second group B of perfume raw materials having a LogP ≥ 4
wherein the sum of perfume raw materials of group A and perfume raw materials of group B is greater than 35% by weight based on the total weight of the perfume raw materials.

Another object of the invention is the use of a composition comprising perfume raw materials;
characterized in that the perfume raw materials comprise:
   - from 0.01% to 50% by weight of a first group A of perfume raw materials having a LogP ≤ 2.5, and
   - from 0.01% to 50% by weight of a second group B of perfume raw materials having a LogP ≥ 4
wherein the sum of perfume raw materials of group A and perfume raw materials of group B is greater than 35% by weight based on the total weight of the perfume raw materials.
to modify the olfactive character of said composition under wet conditions.

Another object of the invention is a method for modifying the olfactive character of a composition comprising the steps consisting of:
(i) providing a perfume composition comprising perfume raw materials;
   characterized in that the perfume raw materials comprises:
      - from 0.01% to 50% by weight of a first group A of perfume raw materials having a LogP ≤ 2.5, and
      - from 0.01% to 50% by weight of a second group B of perfume raw materials having a LogP ≥ 4
   wherein the sum of perfume raw materials of group A and perfume raw materials of group B is greater than 35% by weight based on the total weight of the perfume raw materials.; and
(ii) subjecting said composition to wet conditions.

The invention will now be described in further detail by way of the following examples wherein the amounts are indicated in % by weight, relative to the weight of the perfume composition.

### Example 1

### Olfactive evaluation of the release of a perfume composition incorporated into a deodorant alcoholic spray upon addition of water

The tests were carried out using a standard deodorant alcoholic spray base. The deodorant alcoholic spray base with the following final composition has been prepared.

**Table 1: Deodorant spray formulation**

| **Ingredient** | **Amount (wt %)** |
|---|---|
| Ethanol 95 % | 90.65 |
| Triclosan¹⁾ | 0.26 |
| Isopropyl miristate | 9.09 |

| | |
|---|---|
| 1) Irgasan^{®} DP 300; trademark and origin : BASF | |

### Preparation of deodorant spray formulation

All the ingredients according to the sequence of the Table 1 were mixed and dissolved. Then the aerosol cans were filled, crimp and the propellant (Aerosol filling: 40% active solution 60% Propane / Butane 2.5 bar) was added.

Samples of 40g with the below fragrances were prepared in the deodorant spray base described above: 0.4grs of fragrance with 15.6grs of deodorant liquid base, and with 24grs of gas (= 43.64 mL).
- Fragrance A (table 2)
- Fragrance B (table 3)
- Fragrance C (table 4)
- Fragrance D (table 5)
- Fragrance E (table 6)
- Comparative fragrance X (table 7)

**Table 2: Composition of fragrance A**

| **Ingredients** | **%** | **cLog P** | **ODT** |
|---|---|---|---|
| LINALYL ACETATE | 0.2 | 4.04 | >2.10⁻³ |
| HEXYLCINNAMIC ALDEHYDE | 23.5 | 4.86 | ≤ 2 × 10⁻³ |
| UNDECALACTONE GAMMA | 1.5 | 3.32 | ≤ 2 × 10⁻³ |
| 4-(4-METHOXYPHENYL)-2-BUTANONE | 0.6 | 0.93 | ≤ 2 × 10⁻³ |
| BUTYL HYDROXYPROPIONATE | 0.2 | < 2.5 | >2.10⁻³ |
| ETHYL BUTYRATE | 0.3 | 2.03 | ≤ 2 × 10⁻³ |
| 4-(1,3-BENZODIOXOL-5-YL)-2-BUTANONE | 0.8 | 1.6 | ≤ 2 × 10⁻³ |
| (-)-(2E)-2-ETHYL-4-[(1R)-2,2,3-TRIMETHYL-3-CYCLOPENTEN-1-YL]-2-BUTEN-1-OL | 0.5 | 4.44 | ≤ 2 × 10⁻³ |
| 2-ETHYL-3-HYDROXY-4(4H)-PYRANONE | 1.5 | 0.76 | ≤ 2 × 10⁻³ |
| 3-ETHOXY-4-HYDROXYBENZALDEHYDE | 2.3 | 1.27 | ≤ 2 × 10⁻³ |
| PENTADECENOLIDE | 13.9 | 5.61 | ≤ 2 × 10⁻³ |
| (+-)-5-ETHYL-4-HYDROXY-2-METHYL-3(2H)-FURANONE (A) + (+-)-2-ETHYL-4-HYDROXY-5-METHYL-3(2H)-FURANONE (B) | 0.2 | 0.75 | ≤ 2 × 10⁻³ |
| 3-METHYLBUTYL 2-METHYLPROPANOATE | 0.3 | 3.17 | >2.10⁻³ |
| CINNAMYL ISOBUTYRATE | 0.1 | 3.76 | >2.10⁻³ |
| ETHYL ISOBUTYRATE | 0.4 | 2.03 | ≤ 2 × 10⁻³ |
| LINALOL | 14.4 | 2.94 | ≤ 2 × 10⁻³ |
| (+-)-2-(4-METHYL-3-CYCLOHEXEN-1-YL)-2-PROPANETHIOL | 0.1 | 4.95 | ≤ 2 × 10⁻³ |
| 6,6-DIMETHOXY-2,5,5-TRIMETHYL-2-HEXENE | 2.5 | 3.9 | >2.10⁻³ |
| ETHYL METHYLPHENYLGLYCIDATE | 0.2 | 2.3 | ≤ 2 × 10⁻³ |
| METHYL 2-((1RS,2RS)-3-OXO-2-PENTYLCYCLOPENTYL)ACETATE | 25.5 | 2.92 | ≤ 2 × 10⁻³ |
| (+-)-(1S,4AR,8S,8AR)-2,2,6,8-TETRAMETHYL-1,2,3,4,4A,5,8,8A-OCTAHYDRO-1-NAPHTHALENOL | 0.3 | 5.24 | ≤ 2 × 10⁻³ |
| PATCHOULI OIL | 0.3 | 3.98 | ≤ 2 × 10⁻³ |
| VANILLIN | 0.4 | 0.72 | ≤ 2 × 10⁻³ |
| LIMONENE | 10.0 | 5.4 | >2.10⁻³ |
| % of raw materials having a LogP ≤ 2.5 | 6.7 | | |
| % of raw materials having a LogP ≥ 4 | 48.5 | | |
| % of raw materials having 2.5< LogP<4 | 44.6 | | |

**Table 3: Composition of fragrance B**

| **Ingredients** | **%** | **cLog P** | **ODT** |
|---|---|---|---|
| HEXYL ACETATE | 3.5 | 3.09 | >2.10⁻³ |
| (Z)-3-HEXENYL ACETATE | 1.2 | 2.62 | >2.10⁻³ |
| ALDEHYDE C8 | 2.1 | 2.94 | ≤ 2 × 10⁻³ |
| ALDEHYDE C10 | 15.0 | 3.99 | ≤ 2 × 10⁻³ |
| (+-)-2-METHYLUNDECANAL | 1.4 | 5.01 | ≤ 2 × 10⁻³ |
| UNDECALACTONE GAMMA | 9.3 | 3.32 | ≤ 2 × 10⁻³ |
| NAPHTHO[2,1-B]FURAN, DODECAHYDRO3A,6,6,9A-TETRAMETHYL | 0.2 | 5.83 | ≤ 2 × 10⁻³ |
| CITRONELLYL NITRILE | 19.5 | 3.03 | >2.10⁻³ |
| VERDYL PROPIONATE | 15.1 | 4.38 | ≤ 2 × 10⁻³ |
| (3E,5Z)-1,3,5-UNDECATRIENE | 0.2 | 5.68 | ≤ 2 × 10⁻³ |
| (+-)-3-(4-METHYL-3-CYCLOHEXEN-1-YL)BUTANAL | 1.7 | 3.51 | ≤ 2 × 10⁻³ |
| (+-)-2,6-DIMETHYL-5-HEPTENAL | 2.2 | 3.15 | ≤ 2 × 10⁻³ |
| (+-)-2-(4-METHYL-3-CYCLOHEXEN-1-YL)-2-PROPANETHIOL | 0.1 | 4.95 | ≤ 2 × 10⁻³ |
| METHYLNAPHTHYLKETONE | 2.0 | 2.5 | ≤ 2 × 10⁻³ |
| ETHYL 2 METHYLBUTYRATE | 2.5 | 2.58 | ≤ 2 × 10⁻³ |
| 1-(5,5-DIMETHYL-1-CYCLOHEXEN-1-YL)-4-PENTEN-1-ONE | 0.5 | 3.89 | ≤ 2 × 10⁻³ |
| PINENE MIXTURE | 1.7 | 5.43 | >2.10⁻³ |
| (4Z)-4-DODECENAL | 0.1 | 4.52 | ≤ 2 × 10⁻³ |
| (+-)-3-PHENYLBUTANAL | 0.7 | 2.34 | ≤ 2 × 10⁻³ |
| 2-TERT-BUTYL-1-CYCLOHEXYL ACETATE | 17.4 | 4.4 | >2.10⁻³ |
| 2-METHOXYNAPHTHALENE | 0.2 | 3.29 | ≤ 2 × 10⁻³ |
| 2,4-DIMETHYL-3-CYCLOHEXENE-1-CARBALDEHYDE | 3.4 | 2.34 | ≤ 2 × 10⁻³ |
| % of raw materials having a LogP ≤2.5 | 6.1 | | |
| % of raw materials having a LogP ≥ 4 | 36.2 | | |
| % of raw materials having 2.5< LogP<4 | 57.7 | | |

**Table 4: Composition of fragrance C**

| **Ingredients** | **%** | **cLog P** | **ODT** |
|---|---|---|---|
| BENZYLDIMETHYLCARBINOL ACETATE | 0.5 | 3.45 | >2.10⁻³ |
| ISOBORNYL ACETATE | 19.0 | 4.13 | >2.10⁻³ |
| LINALYL ACETATE | 0.6 | 4.04 | >2.10⁻³ |
| ALDEHYDE C10 | 12.0 | 3.99 | ≤ 2 × 10⁻³ |
| ALDEHYDE C12 | 3.0 | 4.94 | ≤ 2 × 10⁻³ |
| ALDEHYDE SUPRA | 0.6 | 4.26 | ≤ 2 × 10⁻³ |
| UNDECALACTONE GAMMA | 0.1 | 3.32 | ≤ 2 × 10⁻³ |
| BENZYLACETONE PURE | 1.0 | 1.79 | ≤ 2 × 10⁻³ |
| 7-METHYL-2H-1,5-BENZODIOXEPIN-3(4H)-ONE | 0.7 | 1.61 | ≤ 2 × 10⁻³ |
| CAMPHOR | 1.6 | 2.5 | >2.10⁻³ |
| CITRONELLYL NITRILE | 5.0 | 3.03 | >2.10⁻³ |
| DODECANENITRILE | 0.2 | 4.63 | >2.10⁻³ |
| 4-CYCLOHEXYL-2-METHYL-2-BUTANOL | 5.1 | 3.93 | >2.10⁻³ |
| VERDYL PROPIONATE | 0.6 | 4.38 | ≤ 2 × 10⁻³ |
| ((-)-(2E)-2-ETHYL-4-[(1R)-2,2,3-TRIMETHYL-3-CYCLOPENTEN-1-YL]-2-BUTEN-1-OL | 0.2 | 4.44 | ≤ 2 × 10⁻³ |
| DIHYDROMYRCENOL | 22.3 | 3.21 | >2.10⁻³ |
| DIMETHYLOCTANOL | 4.2 | 4.24 | >2.10⁻³ |
| (2RS,6RS)-2,4,6-TRIMETHYL-4-PHENYL-1,3-DIOXANE | 0.2 | 2.9 | >2.10⁻³ |
| GERANIOL PUR | 0.5 | 2.97 | ≤ 2 × 10⁻³ |
| PENTADECENOLIDE | 0.2 | 5.61 | ≤ 2 × 10⁻³ |
| 3,7-DIMETHYL-2,6-NONADIENENITRILE (A) + 3,7-DIMETHYL-3,6-NONADIENENITRILE (B) | 8.5 | 3.17 | ≤ 2 × 10⁻³ |
| (+-)-3-(4-METHYL-3-CYCLOHEXEN-1-YL)BUTANAL | 1.0 | 3.51 | ≤ 2 × 10⁻³ |
| LINALOL | 0.4 | 2.94 | ≤ 2 × 10⁻³ |
| METHYL 2-((1RS,2RS)-3-OXO-2-PENTYLCYCLOPENTYL)ACETATE | 1.3 | 2.92 | ≤ 2 × 10⁻³ |
| (+-)-TETRAHYDRO-2-ISOBUTYL-4-METHYL-4(2H)-PYRANOL | 1.4 | 2.22 | >2.10⁻³ |
| (+)-(3R)-1-[(1R,6S)-2,2,6-TRIMETHYLCYCLOHEXYL]-3-HEXANOL | 0.4 | 5.52 | ≤ 2 × 10⁻³ |
| ETHYL OENANTHATE | 0.9 | 3.58 | >2.10⁻³ |
| (2RS,4SR)-4-METHYL-2-(2-METHYL-1-PROPEN-1-YL)TETRAHYDRO-2H-PYRAN (A) + (2RS,4RS)-4-METHYL-2-(2-METHYL-1-PROPEN-1-YL)TETRAHYDRO-2H-PYRAN (B) | 0.1 | 3.49 | ≤ 2 × 10⁻³ |
| PHENYLETHYL ALCOHOL | 2.1 | 1.41 | ≤ 2 × 10⁻³ |
| (Z)-3-HEXEN-1-OL | 0.2 | 1.65 | >2.10⁻³ |
| 9-DECEN-1-OL | 0.1 | 3.7 | >2.10⁻³ |
| (-)-PROPYL (S)-2-(1,1-DIMETHYLPROPOXY)PROPANOATE | 0.5 | 3.17 | >2.10⁻³ |
| TERPINEOL | 1.8 | 2.91 | >2.10⁻³ |
| 2-TERT-BUTYL-1-CYCLOHEXYL ACETATE | 3.1 | 4.4 | >2.10⁻³ |
| 2-METHOXYNAPHTHALENE | 0.3 | 3.29 | ≤ 2 × 10⁻³ |
| 2,4-DIMETHYL-3-CYCLOHEXENE-1-CARBALDEHYDE | 0.3 | 2.34 | ≤ 2 × 10⁻³ |
| % of raw materials having a LogP ≤2.5 | 7.3 | | |
| % of raw materials having a LogP ≥ 4 | 32.1 | | |
| % of raw materials having 2.5< LogP<4 | 60.6 | | |

**Table 5: Composition of fragrance D**

| **Ingredients** | **%** | **cLog P** | **ODT** |
|---|---|---|---|
| BENZYL ACETATE | 7.0 | 2.04 | >2.10⁻³ |
| PHENYLETHYL ACETATE | 3.6 | 2.49 | >2.10⁻³ |
| PHENYLPROPYL ALCOHOL | 1.0 | 1.81 | >2.10⁻³ |
| 2-METHYL-4-PHENYL-2-BUTANOL | 2.5 | 2.69 | >2.10⁻³ |
| NAPHTHO[2,1-B]FURAN, DODECAHYDRO3A,6,6,9A-TETRAMETHYL | 2.0 | 5.83 | ≤ 2 × 10⁻³ |
| 7-HYDROXY-2-CHROMENONE | 0.6 | 1.35 | ≤ 2 × 10⁻³ |
| OXACYCLOHEXADECAN-2-ONE | 6.0 | 7.2 | ≤ 2 × 10⁻³ |
| DAMASCONE ALPHA | 0.8 | 3.65 | ≤ 2 × 10⁻³ |
| ETHYL DAMASCENATE | 1.2 | 3.16 | >2.10⁻³ |
| (+-)-(1-ETHOXYETHOXY)CYCLODODECANE | 2.5 | 6.68 | >2.10⁻³ |
| ETHYL 2-METHYL-1,3-DIOXOLANE-2-ACETATE | 7.0 | 1.12 | ≤ 2 × 10⁻³ |
| GERANIOL PUR | 8.0 | 2.97 | ≤ 2 × 10⁻³ |
| 3-(1,3-BENZODIOXOL-5-YL)-2-METHYLPROPANAL | 1.0 | 1.28 | ≤ 2 × 10⁻³ |
| 3-(3,3-DIMETHYL-2,3-DIHYDRO-1H-INDEN-5-YL)PROPANAL (A) + 3-(1,1-DIMETHYL-2,3-DIHYDRO-1H-INDEN-4-YL)PROPANAL (B) + 3-(1,1-DIMETHYL-2,3-DIHYDRO-1H-INDEN-5-YL)PROPANAL (C) | 0.2 | 3.44 | ≤ 2 × 10⁻³ |
| 1-(2,2,3,6-TETRAMETHYL-CYCLOHEXYL)-3-HEXANOL | 2.5 | 5.96 | ≤ 2 × 10⁻³ |
| METHYLISOEUGENOL | 2.0 | 2.85 | >2.10⁻³ |
| (+-)-(4E)-3-METHYL-4-CYCLOPENTADECEN-1-ONE (A) + (+-)-(5E)-3-METHYL-5-CYCLOPENTADECEN-1-ONE (B) + (+-)-(5Z)-3-METHYL-5-CYCLOPENTADECEN-1-ONE (C) | 0.8 | 5.98 | ≤ 2 × 10⁻³ |
| ETHYL 2 METHYLBUTYRATE | 0.8 | 2.58 | ≤ 2 × 10⁻³ |
| (+-)-TETRAHYDRO-2-ISOBUTYL-4-METHYL-4(2H)-PYRANOL | 4.0 | 2.22 | >2.10⁻³ |
| PHENYLETHYL ALCOHOL | 23.0 | 1.41 | ≤ 2 × 10⁻³ |
| (Z)-3-HEXEN-1-OL | 1.0 | 1.65 | >2.10⁻³ |
| (+-)-3,7-DIMETHYL-3-OCTANOL | 17.0 | 3.78 | >2.10⁻³ |
| 3-(DODECYLTHIO)-1-[(1RS,2SR)-2,6,6-TRIMETHYL-3-CYCLOHEXEN-1-YL]-1-BUTANONE | 1.8 | >8 | >2.10⁻³ |
| 3-(4,4-DIMETHYL-1-CYCLOHEXEN-1-YL)PROPANAL | 0.8 | 3.3 | >2.10⁻³ |
| (3E)-4-(2,6,6-TRIMETHYL-1-CYCLOHEXEN-1-YL)-3-BUTEN-2-ONE | 2.5 | 3.88 | ≤ 2 × 10⁻³ |
| 2,4-DIMETHYL-3-CYCLOHEXENE-1-CARBALDEHYDE | 0.4 | 2.34 | ≤ 2 × 10⁻³ |
| % of raw materials having a LogP ≤2.5 | 48.6 | | |
| % of raw materials having a LogP ≥4 | 15.6 | | |
| % of raw materials having 2.5< LogP<4 | 35.8 | | |

**Table 6: Composition of fragrance E**

| **Ingredients** | **%** | **cLog P** | **ODT** |
|---|---|---|---|
| BENZYL ACETATE | 0.4 | 2.04 | >2.10⁻³ |
| ISOBORNYL ACETATE PURE | 5.9 | 4.13 | >2.10⁻³ |
| (+-)-1-PHENYLETHYL ACETATE | 0.8 | 2.22 | >2.10⁻³ |
| ANISALDEHYDE | 0.8 | 1.56 | ≤ 2 X 10⁻³ |
| ALDEHYDE C10 | 0.1 | 3.99 | ≤ 2 X 10⁻³ |
| (+-)-2-METHYLUNDECANAL | 1.7 | 5.01 | ≤ 2 X 10⁻³ |
| ALDEHYDE SUPRA | 0.7 | 4.26 | ≤ 2 X 10⁻³ |
| (3AR,5AS,9AS,9BR)-3A,6,6,9A-TETRAMETHYLDODECAHYDRONAPHTHO[2, 1-B]FURAN | 0.2 | 6.85 | ≤ 2 X 10⁻³ |
| UNDECALACTONE GAMMA | 0.8 | 3.32 | ≤ 2 X 10⁻³ |
| BENZYLACETONE PURE | 1.0 | 1.79 | ≤ 2 X 10⁻³ |
| 2-ETHOXYNAPHTHALENE | 0.4 | 3.82 | >2.10⁻³ |
| CITRONELLOL | 0.6 | 3.37 | ≤ 2 X 10⁻³ |
| 4-CYCLOHEXYL-2-METHYL-2-BUTANOL | 1.3 | 3.93 | >2.10⁻³ |
| VERDYL ACETATE | 4.5 | 3.73 | ≤ 2 X 10⁻³ |
| (+-)-4-METHYLENE-2-PHENYLTETRAHYDRO-2H-PYRAN (A) + (+-)-4-METHYL-6-PHENYL-3,6-DIHYDRO-2H-PYRAN (B) + (+-)-4-METHYL-2-PHENYL-3,6-DIHYDRO-2H-PYRAN (C) | 0.2 | 3.33 | ≤ 2 X 10⁻³ |
| DIHYDROMYRCENOL PURE | 25.2 | 3.21 | >2.10⁻³ |
| EUCALYPTOL | 0.8 | 3.31 | ≤ 2 X 10⁻³ |
| 2-METHYL-4-[(1R)-2,2,3-TRIMETHYL-3-CYCLOPENTEN-1-YL]-4-PENTEN-1-OL | 0.3 | 4.43 | ≤ 2 X 10⁻³ |
| (+-)-(3E)-3-METHYL-4-(2,6,6-TRIMETHYL-2-CYCLOHEXEN-1-YL)-3-BUTEN-2-ONE (A) + (+-)-(1E)-1-(2,6,6-TRIMETHYL-2-CYCLOHEXEN-1-YL)-1-PENTEN-3-ONE (B) | 0.6 | 4.28 | ≤ 2 X 10⁻³ |
| (3ARS,6SR,7ASR)-PERHYDRO-3,6-DIMETHYL-BENZO[B]FURAN-2-ONE | 0.1 | 2.14 | ≤ 2 X 10⁻³ |
| (+-)-2-METHYL-3-[4-(2-METHYL-2-PROPANYL)PHENYL]PROPANAL | 2.0 | 3.9 | ≤ 2 X 10⁻³ |
| LINALOL | 0.2 | 2.94 | ≤ 2 X 10⁻³ |
| (-)-(1R,8S)-2,2,7,7-TETRAMETHYLTRICYCLO[6.2.1.0∼1,6∼]UNDE C-5-ENE | 1.6 | 5.4 | >2.10⁻³ |
| MENTHOL | 0.4 | 3.42 | >2.10⁻³ |
| MENTHONE | 0.1 | 3.46 | >2.10⁻³ |
| 3-MÉTHYL-CYCLOPENTADÉCANONE | 0.2 | 6.11 | ≤ 2 X 10⁻³ |
| METHYL 2-((1RS,2RS)-3-OXO-2-PENTYLCYCLOPENTYL)ACETATE | 11.3 | 2.92 | ≤ 2 X 10⁻³ |
| ETHYL 2 METHYLBUTYRATE | 0.7 | 2.58 | ≤ 2 X 10⁻³ |
| PATCHOULI OIL | 1.3 | 3.98 | ≤ 2 X 10⁻³ |
| PHENYLETHYL ALCOHOL | 6.9 | 1.41 | ≤ 2 X 10⁻³ |
| AMYL SALICYLATE | 1.1 | 4.82 | >2.10⁻³ |
| CYCLOHEXYL SALICYLATE | 1.9 | 5.32 | >2.10⁻³ |
| (2Z)-2-PHENYL-2-HEXENENITRILE | 2.6 | 3.29 | >2.10⁻³ |
| (+-)-3,7-DIMETHYL-3-OCTANOL | 4.0 | 3.78 | >2.10⁻³ |
| 1-(2,3,8,8-TETRAMETHYL-1,2,3,4,6,7,8,8A-OCTAHYDRO-2-NAPHTHALENYL)ETHANONE (A) + 1-(2,3,8,8-TETRAMETHYL-1,2,3,5,6,7,8,8A-OCTAHYDRO-2-NAPHTHALENYL)ETHANONE (B) + 1-(2,3,8,8-TETRAMETHYL-1,2,3,4,5,6,7,8-OCTAHYDRO-2-NAPHTHALENYL)ETHANONE (C) | 2.2 | 5.82 | ≤ 2 X 10⁻³ |
| 2-TERT-BUTYL-1-CYCLOHEXYL ACETATE | 16.3 | 4.4 | >2.10⁻³ |
| 2-METHOXYNAPHTHALENE | 0.2 | 3.29 | ≤ 2 X 10⁻³ |
| 2,4-DIMETHYL-3-CYCLOHEXENE-1-CARBALDEHYDE | 0.6 | 2.34 | ≤ 2 X 10⁻³ |
| % of raw materials having a LogP ≤ 2.5 | 10.6 | | |
| % of raw materials having a LogP ≥4 | 32.7 | | |
| % of raw materials having 2.5< LogP<4 | 56.7 | | |

**Table 7: Comparative fragrance X formulation**

| Raw materials | % | cLog P | ODT |
|---|---|---|---|
| HEXYL ACETATE | 4.5% | 3.09 | >2.10⁻³ |
| 2,6-DIMETHYL-4-HEPTANOL | 5.0% | 3.08 | >2.10⁻³ |
| DIHYDROMYRCENOL | 20.8% | 3.21 | >2.10⁻³ |
| PHENETHYLOL | 16.9% | 1.41 | ≤ 2 X 10⁻³ |
| (+-)-3,7-DIMETHYL-3-OCTANOL | 23.5% | 3.78 | >2.10⁻³ |
| BENZYL ACETATE | 1.8% | 2.04 | >2.10⁻³ |
| (+-)-1-PHENYLETHYL ACETATE | 0.7% | 2.22 | >2.10⁻³ |
| ALPHA TERPINEOL | 2.4% | 2.91 | >2.10⁻³ |
| PHENOXY | 1.5% | 1.14 | >2.10⁻³ |
| (+-)-TETRAHYDRO-2-ISOBUTYL-4-METHYL-4(2H)-PYRANOL | 2.6% | 2.22 | >2.10⁻³ |
| (-)-PROPYL (S)-2-(1,1-DIMETHYLPROPOXY)PROPANOATE | 4.3% | 3.17 | >2.10⁻³ |
| [4-(2-PROPANYL)CYCLOHEXYL] | 4.6% | 3.45 | >2.10⁻³ |
| 2-METHYL-4-PHENYL-2-BUTANOL | 0.9% | 2.69 | >2.10⁻³ |
| 2-TERT-BUTYL-1-CYCLOHEXYL ACETATE | 6.8% | 4.4 | >2.10⁻³ |
| PHENYLHEXANOL | 2.0% | 3 | ≤ 2 X 10⁻³ |
| AMYLE SALICYLATE | 1.2% | 4.82 | >2.10⁻³ |
| 2-{(1S)-1-[(1R)-3,3-DIMETHYLCYCLOHEXYL]ETHOXY}-2-OXOETHYL PROPIONATE | 0.5% | 4.56 | >2.10⁻³ |
| % of raw materials having a LogP ≤2.5 | 23.5 | | |
| % of raw materials having a LogP≥ 4 | 8.5 | | |
| % of raw materials having 2.5< LogP<4 | 68 | | |

The samples were freshly produced for the evaluation.

0.35g of sample was applied on a cardboard blotter (4.5cm* 12 cm): 2 blotters were prepared by sample.

After 6 hours of drying, panelists assessed the olfactive intensity, using a scale from "1" (no odor) to "7" (very strong), and described olfactively the hedonics of the fragrance.

The results are below.

**Table 8: Olfactive performance results**

| | Blotter without water | | Blotter after spraying 0.3g of water | |
|---|---|---|---|---|
| Fragrance | Olfactive Intensity | Olfactive description | Olfactive Intensity | Olfactive description |
| Comparative fragrance X | 4 | Floral Green Woody | 4 | Floral Green Woody |
| Fragrance A | 5 | Fruity Strawberry Vanilla | 6.5 | Fruity Pear Vanilla Chocolate |
| Fragrance B | 5.5 | Citrus Aldehydic Musky | 6 | Orange Lime Aldehydic |
| Fragrance C | 6 | Citrus Aldehydic Musky | 6.5 | Watery Cucumber Woody |
| Fragrance D | 4 | Floral Rose Fruity Green | 5 | Fruity Green Powdery Musky |
| Fragrance E | 5 | Floral Powdery Aldehydic Musky | 6 | Floral Aromatic Lavender |

The evaluation showed significant differences in intensity and a change of hedonics after the addition of water for the perfume compositions according to the invention.

Comparative fragrance X does not show any significant change.

### Example 2

### Olfactive evaluation of the release of a fragrance incorporated into an anti-perspirant spray upon addition of water

The tests were carried out using a standard anti-perspirant spray base. The anti-perspirant spray base with the following final composition has been prepared.

**Table 9: Antiperspirant spray anhydrous formulation**

| **Ingredient** | **Amount (wt %)** |
|---|---|
| Cyclomethicone ¹⁾ | 53.51 |
| Isopropyl miristate | 9.04 |
| Silica²⁾ | 1.03 |
| Quaternium-18-Hectorite³⁾ | 3.36 |
| Aluminium Chlorohydrate⁴⁾ | 33.06 |

| | |
|---|---|
| 1) Dow Corning^{®} 345 Fluid; trademark and origin: Dow Corning 2) Aerosil^{®} 200 ; trademark and origin : Evonik 3) Bentone^{®} 38; trademark and origin : Elementis Specialities 4) Micro Dry Ultrafine; origin : Reheis | |

### Preparation of the anti-perspirant spray formulation

Using a high speed stirrer, Silica and Quaternium-18-Hectorite were added to the Isopropyl miristate and Cyclomethicone mixture. Once completely swollen, Aluminium Chlorohydrate was added portion wise under stirring until the mixture was homogeneous and without lumps. The aerosol cans were filled with 25 % Suspension of the suspension and 75 % of Propane/Butane (2,5 bar).

Samples of 40g with the below fragrances were prepared in the anti-perspirant spray base described above: 0.4grs of fragrance with 9.6grs of deodorant liquid base, and with 30grs of gas (= 54.55 mL).
- Fragrance A
- Fragrance D

The samples were freshly produced for the evaluation.

0.25g of sample was applied on a cardboard blotter (4.5cm* 12 cm): 2 blotters were prepared by sample.

After 6 hours of drying, panelists assessed the olfactive intensity, using a scale from "1" (no odor) to "7" (very strong), and described olfactively the hedonics of the fragrance.

The results are below.

**Table 10: Olfactive performance results**

| | Blotter without water | | Blotter after spraying 0.3g of water | |
|---|---|---|---|---|
| **Fragrance** | **Olfactive Intensity** | **Olfactive description** | **Olfactive Intensity** | **Olfactive description** |
| Comparative fragrance X | 4 | Woody Aromatic Amber | 4 | Woody Aromatic Amber |
| Fragrance A | 5.5 | Fruity Strawberry Vanilla | 6 | Fruity Pear Vanilla Chocolate |
| Fragrance D | 5.5 | Floral Rose Fruity Green | 6 | Fruity Green Powdery Musky |

The evaluation showed significant differences in intensity and a change of hedonics after the addition of water for the perfume compositions according to the invention.

Comparative fragrance X does not show any significant change.

### Example 3

### Antiperspirant spray emulsion compositions

Fragrance A-E (A or B or C or D or E) is weighed and mixed in an antiperspirant spray emulsion composition (see Table 11).

**Table 11: antiperspirant spray emulsion composition**

| **Ingredient** | **Amount (wt %)** |
|---|---|
| Polysorbate 65¹⁾ (Part A) | 0.95 |
| Polyglyceryl-2 dipolyhydroxystearate²⁾ (Part A) | 1.05 |
| Cetyl PEG/PPG-10/1 Dimethicone³⁾ (Part A) | 2.75 |
| Cyclomethicone⁴⁾ (Part A) | 16.4 |
| Isopropylisostearate⁵⁾ (Part A) | 4.5 |
| Phenoxyethanol⁶⁾ (Part A) | 0.5 |
| Ethylhexylglycerin⁷⁾ (Part A) | 0.2 |
| C12-15 Alkyl Benzoate⁸⁾ (Part A) | 5.65 |
| Silica Silylatey⁹⁾ (Part A) | 0.1 |
| Sodium Methylparaben¹⁰⁾ (Part B) | 0.1 |
| Aluminium Chlorohydrate¹¹⁾ (Part B) | 20 |
| Water (Part B) | 44.47 |
| Fragrance A-E (Part C) | 3.33 |

| | |
|---|---|
| 1) Tween 65; trademark and origin : CRODA 2) Dehymuls PGPH; trademark and origin : BASF 3) Abil EM-90; trademark and origin : BASF 4) Dow Corning 345 fluid; trademark and origin : Dow Corning 5) Crodamol ipis; trademark and origin : CRODA 6) Phenoxyethanol; trademark and origin : LANXESS 7) Sensiva sc 50; trademark and origin : KRAFT 8) Tegosoft TN; trademark and origin : Evonik 9) Aerosil R 812; trademark and origin : Evonik 10) Nipagin mna; trademark and origin : CLARIANT 11) Locron L; trademark and origin : CLARIANT | |

The ingredients of Part A and Part B are weighted separately. Ingredients of Part A are heated up to 60°C and ingredients of Part B are heated to 55 °C. Ingredients of Part B are poured small parts while continuous stirring into A. Mixture were stirred well until the room temperature was reached. Then, ingredients of part C are added. The emulsion is mixed and is introduced into the aerosol cans. The propellant is crimped and added. Aerosol filling: 30% Emulsion: 70% Propane / Butane 2,5 bar

### Example 4

### Antiperspirant roll-on emulsion composition

Fragrance A-E is weighed and mixed in an antiperspirant composition (see Table 12).

**Table 12: antiperspirant roll-on emulsion composition**

| **Ingredient** | **Amount (wt %)** |
|---|---|
| Steareth-2¹⁾ (Part A) | 3.25 |
| Steareth-21²⁾ (Part A) | 0.75 |
| PPG-15 Stearyl Ether³⁾ (Part A) | 4 |
| WATER deionised (Part B) | 51 |
| Aluminum Chlorohydrate 50% aqueous solution⁴⁾ (Part C) | 40 |
| Fragrance A-E (Part D) | 1 |

| | |
|---|---|
| 1) BRIJ 72; origin : ICI 2) BRIJ 721; origin : ICI 3) ARLAMOL E; origin : UNIQEMA-CRODA 4) LOCRON L; origin : CLARIAN | |

Part A and B are heated separately to 75°C; Part A is added to part B under stirring and the mixture is homogenized for 10 minutes. Then, the mixture is cooled down under stirring; and part C is slowly added when the mixture reached 45°C and part D when the mixture reached at 35 °C while stirring. Then the mixture is cooled down to RT.

### Example 5

### Antiperspirant roll-on composition

Fragrance A-E is weighed and mixed in an antiperspirant composition (see Table 13).

**Table 13: antiperspirant roll-on composition**

| **Ingredient** | **QUANTITY** |
|---|---|
| Water (Part A) | 45 |
| Aluminum Chlorohydrate 50% aqueous solution¹⁾ (Part B) | 20 |
| Alcohol Denat. (Ethanol 96%) (Part B) | 30 |
| Ceteareth-12²⁾ (Part C) | 2 |
| Ceteareth-30³⁾ (Part C) | 2 |
| Fragrance A-E (Part D) | 1 |

| | |
|---|---|
| 1) LOCRON L; origin: CLARIANT 2) EUMULGIN B-1; origin : BASF 3) EUMULGIN B-3; origin : BASF | |

The ingredients of part B are mixed in the vessel then ingredient of part A is added. Then dissolved part C in part A and B. With perfume, 1 part of Cremophor RH40 for 1 part of perfume is added while mixing well.

### Example 6

### Antiperspirant roll-on composition

A sufficient amount of fragrance A-E is weighed and mixed in an antiperspirant composition (see Table 14) to add the equivalent of 1% perfume.

**Table 14: antiperspirant roll-on emulsion composition**

| **Ingredient** | **Amount (wt %)** |
|---|---|
| Water (Part A) | 50.51 |
| Hydroxyethylcellulose¹⁾ (Part A) | 0.71 |
| Ethanol 95 % (Part B) | 40.40 |
| 1,2-Propylene Glycol (Part B) | 5.05 |
| Triclosan²⁾ (Part B) | 0.30 |
| PEG-40 Hydrogenated castor oil³⁾ (Part C) | 3.03 |

| | |
|---|---|
| 1) Natrosol^{®} 250 H; trademark and origin: Ashland 2) Irgasan^{®} DP 300; trademark and origin : BASF 3) Cremophor^{®} RH 40; trademark and origin : BASF | |

Part A is prepared by sprinkling little by little the hydroxyethylcellulose in the water whilst rapidly stirring with the turbine. Stirring is continued until the hydroxyethylcellulose is entirely swollen and giving a limpid gel. Then, Part B is poured little by little in Part A whilst continuing stirring until the whole is homogeneous. Part C is added.

### Example 7

### Deodorant pump without alcohol formulation

Fragrance A-E is weighed and mixed in a deodorant composition (see Table 15).

**Table 15: deodorant composition**

| **Ingredients** | **Amount (wt %)** |
|---|---|
| C12-15 Alkyl Lactate¹⁾ | 5 |
| Dimethicone²⁾ | 91.6 |
| Cetyl Lactate³⁾ | 1 |
| Octyldodecanol⁴⁾ | 0.8 |
| Triclosan⁵⁾ | 0.1 |
| Fragrance A-E | 1.5 |

| | |
|---|---|
| 1) Ceraphyl 41; trademark and origin ASHLAND 2) DOW CORNING 200 FLUID 0.65cs; trademark and origin DOW CORNING CORPORATION 3) Ceraphyl 28; trademark and origin ASHLAND 4) Eutanol G; trademark and origin BASF 5) Irgasan^{®} DP 300; trademark and origin : BASF | |

All the ingredients are mixed according to the sequence of the table and the mixture is heated slightly to dissolve the Cetyl Lactate.

### Example 8

### Deodorant pump with alcohol formulation

Fragrance A-E is weighed and mixed in a deodorant composition (see Table 16).

**Table 16: deodorant composition**

| **Ingredients** | **Amount (wt %)** |
|---|---|
| Ethyl Alcohol (Part A) | 60 |
| PEG-6 Caprylic/Capric Glycerides¹⁾ (Part A) | 2 |
| Water (Part A) | 35.6 |
| PEG-40 Hydrogenated Castor Oil²⁾ (Part B) | 0.4 |
| Fragrance A-E (Part B) | 2 |

| | |
|---|---|
| 1) Softigen 767; trademark and origin CRODA 2) Cremophor^{®} RH 40; trademark and origin : BASF | |

Ingredients from Part B are mixed together. Ingredients of Part A are dissolved according to the sequence of the Table and are poured into part B.

### Example 9

### Deodorant stick without alcohol formulation

A sufficient amount of fragrance A-E is weighed and mixed in a deodorant composition (see Table 17) to add the equivalent of 1% perfume.

**Table 17: deodorant composition**

| **Ingredient** | **Amount (wt %)** |
|---|---|
| Stearic acid (Part A) | 5.05 |
| 1,2-propylene glycol (Part A) | 41.87 |
| Sodium hydroxide 20% aqueous solution (Part A) | 4.24 |
| Water (Part A) | 30.30 |
| Tetrasodium EDTA¹⁾ (Part A) | 0.10 |
| Ceteareth-25²⁾ (Part A) | 1.52 |
| PPG-3 Myristyl ether³⁾ (Part A) | 1.52 |
| 1,2-propylene glycol (Part B) | 15.14 |
| Triclosan⁴⁾ (Part B) | 0.25 |

| | |
|---|---|
| 1) Edeta^{®} B Power; trademark and origin : BASF 2) Cremophor^{®} A25; trademark and origin: BASF 3) Tegosoft^{®} APM; trademark and origin: Evonik 4) Irgasan^{®} DP 300; trademark and origin : BASF | |

All the components of Part A are weighted and heated up to 70-75°C. Ceteareth-25 is added once the other Part A ingredients are mixed and heated. Once the Ceteareth-25 is dissolved, the Stearic Acid is added. Part B is prepared by dissolving the Triclosan in 1,2 Propylene Glycol. Water which has evaporated is added. Slowly under mixing, Part B is poured into part A. To stock, a plastic bag into the bucket is put in to be sealed after cooling. Mould was filled at about 70°C.

### Example 10

### Anti-perspirant stick

A sufficient amount of fragrance A-E is weighed and mixed in antiperspirant composition (see Table 18) to add the equivalent of 1% perfume.

**Table 18: antiperspirant composition**

| **Ingredient** | **Amount (wt %)** |
|---|---|
| Cyclomethicone¹⁾ (Part A) | 55.56 |
| Stearyl Alcohol²⁾ (Part A) | 21.21 |
| PPG-14 Butyl ether³⁾ (Part A) | 2.02 |
| Hydrogenated Castor Oil⁴⁾ (Part A) | 1.01 |
| Aluminium Zirconium tetrachlorohydrex-Gly⁵⁾ (Part B) | 20.20 |

| | |
|---|---|
| 1) Dow Corning^{®} 345 Fluid; trademark and origin: Dow Corning 2) Lanette^{®} 18; trademark and origin: BASF 3) Tegosoft^{®} PBE; trademark and origin: Evonik 4) Cutina^{®} HR; trademark and origin: BASF 5) Summit AZP-908; trademark and origin: Reheis | |

All the components of Part A are weighted, heated up to 70-75°C and mixed well. Ingredient of Part B is dispersed in Part A. The mixture is mixed and putted into a tick at 65 °C.

## Claims

1. An antiperspirant or deodorant composition comprising:
- an antiperspirant or deodorant active material;
- optionally a carrier; and
- a perfume composition comprising perfume raw materials;
**characterized in that** the perfume raw materials comprises
- from 0.01% to 50% by weight of a first group A of perfume raw materials having a LogP ≤ 2.5, and
- from 0.01% to 50% by weight of a second group B of perfume raw materials having a LogP ≥ 4
wherein the sum of perfume raw materials of group A and perfume raw materials of group B is greater than 35% by weight based on the total weight of the perfume raw materials.

2. The composition according to claim 1, wherein the first group of perfume raw materials comprises perfume raw material having a LogP ≤ 2.5 and the second group of perfuming compounds comprises perfume raw materials having a LogP ≥ 4.5.

3. The composition according to claim 1 or 2, wherein the perfume raw materials comprise between 2-25% by weight of the first group A of perfume raw materials and/or between 2-25% by weight of the second group B of perfume raw materials.

4. The composition according to anyone of the preceding claims, wherein the sum of perfume raw materials of group A and perfume raw materials of group B having an odor detection threshold (ODT) ≤ 2 × 10⁻³ µg/L is greater than 8 % by weight based on the total weight of the perfume raw materials.

5. The composition according to anyone of claims 1-4, wherein perfume raw materials from the first group A are chosen in the group consisting of 2-(4-methyl-1,3-thiazol-5-yl)-1-ethanol, 3-ethoxy-4-hydroxybenzaldehyde, 2-ethyl-3-hydroxy-4(4H)-pyranone, 4-(4-hydroxyphenyl)-2-butanone, 7-hydroxy-2-chromenone, phenyl ethyl alcohol, aldehyde anisique, cinnamic alcohol, 4-nonanolide, 2-methoxy-4-(2-propen-1-yl)phenol, methyl 2,4-dihydroxy-3,6-dimethylbenzoate, phenyl ethyl acetate, vanillin, isoeugenol, (3aRS,6SR,7aSR)-perhydro-3,6-dimethyl-benzo[b]furan-2-one, ethyl butyrate, methyl naphtyl ketone, 7-methyl-2H-1,5-benzodioxepin-3(4H)-one , 3-(1,3-benzodioxol-5-yl)-2-methylpropanal, 2,4-dimethyl-3-cyclohexene-1-carbaldehyde, (+-)-3-phenylbutanal, benzylacetone, 4-(3,4-methylenedioxyphenyl)-2-butanone, 5-ethyl-4-hydroxy-2-methyl-furan-3-one, ethyl isobutyrate, ethyl methylphenylglycidate, ethyl 2-methyl-1,3-dioxolane-2-acetate, benzenemethanol, alpha-methylene, acetate, and mixtures thereof.

6. The composition according to anyone of claims 1-4, wherein perfume raw materials from the second group are chosen in the group consisting of (4Z)-4-dodecenal, 3,3-dimethyl-5-(2,2,3-trimethyl-3-cyclopenten-1-yl)-4-penten-2-ol,{1-methyl-2-[(1,2,2-trimethylbicyclo[3.1.0]hex-3-yl)methyl]cyclopropyl}methanol, 2-methylundecanal, (1S,1'R)-2-[1-(3',3'-dimethyl-1'-cyclohexyl)ethoxy]-2-methylpropyl propanoate, (3aR,5aS,9aS,9bR)-3a,6,6,9a-tetramethyldodecahydronaphtho[2,1-b]furan, 1-(2,2,3,6-tetramethyl-cyclohexyl)-3-hexanol, 1-(octahydro-2,3,8,8-tetramethyl-2-naphtalenyl)-1-ethanone, (2e)-tridec-2-enenitrile, verdyl propionate, naphtho[2,1-b]furan, dodecahydro3a,6,6,9a-tetramethyl, patchoulol, aldehyde C12, 1-[(1RS,6SR)-2,2,6-trimethylcyclohexyl]-3-hexanol, (+)-(3R)-1-[(1R,6S)-2,2,6-trimethylcyclohexyl]-3-hexanol, (3E,5Z)-1,3,5-undecatriene, pentadecenolide, (+-)-2-(4-methyl-3-cyclohexen-1-yl)-2-propanethiol, aldeyhyde supra, ((-)-(2e)-2-ethyl-4-[(1R)-2,2,3-trimethyl-3-cyclopenten-1-yl]-2-buten-1-ol, 2-methyl-4-(2,2,3- trimethyl-3-cyclopenten-1-yl)-4-penten-1-ol, methyl-ionone, 3-methyl-cyclopentadecanone, aldehyde hexylcinnamique, (+-)-(1S,4aR,8S,8aR)-2,2,6,8-tetramethyl-1,2,3,4,4a,5,8,8a-octahydro-1-naphthalenol, oxacyclohexadecan-2-one, muscenone delta, delta damascene and mixtures thereof.

7. The composition according to anyone of the preceding claims, wherein the carrier is liquid and selected in the group consisting of ethanol, water, a surfactant, and mixtures thereof.

8. The composition according to anyone of the claims 1 to 6, wherein the composition is anhydrous.

9. The composition according to anyone of the preceding claims, wherein the perfume composition comprises in addition to perfume raw materials a solvent chosen in the group consisting of dipropylene glycol, Isopar M (hydrocarbons C13-C14), Isopar L (hydrocarbons C11 - C13), isopropyl myristate (isopropyl tetradecanoate) ethyle citrate (triethyl 2-hydroxy-1,2,3-propanetricarboxylate), triacetine (1,2,3-propanetriyl triacetate), 1,3-propanediol, mixture of methyl dihydroabietate and methyl tetrahydroabietate, vegetable oils such as almond oil, argan oil, cotton oil, corn oil, olive oil, sunflower oil, castor oil and mixtures thereof.

10. The composition according to anyone of the preceding claims, wherein the perfume composition further comprises at least 10% by weight of perfume raw materials of group C having a log P comprised between 2.5 and 4 and/or an odor detection threshold (ODT) of ≤ 2 × 10⁻³ µg/L, preferably chosen in the group consisting of linalyl acetate, benzyl benzoate, dihydromyrcenol, linalol, sclareolate, ethyl acetoacetate, and mixtures thereof.

11. The composition according to anyone of the preceding claims, wherein the antiperspirant or deodorant is chosen in the group consisting of a body spray formulation, a solid formulation, a roll-on formulation and an aerosol formulation.

12. A perfume composition comprising perfume raw materials;
**characterized in that** the perfume raw materials comprises:
- from 0.01% to 50% by weight of a first group A of perfume raw materials having a LogP ≤ 2.5, and
- from 0.01% to 50% by weight of a second group B of perfume raw materials having a LogP ≥ 4
wherein the sum of perfume raw materials of group A and perfume raw materials of group B is greater than 35% by weight based on the total weight of the perfume raw materials.

13. Use of a composition comprising perfume raw materials;
**characterized in that** the perfume raw materials comprises:
- from 0.01% to 50% by weight of a first group A of perfume raw materials having a LogP ≤ 2.5, and
- from 0.01% to 50% by weight of a second group B of perfume raw materials having a LogP ≥ 4
wherein the sum of perfume raw materials of group A and perfume raw materials of group B is greater than 35% by weight based on the total weight of the perfume raw materials to modify the olfactive character of said composition under wet conditions.

14. A method for modifying the olfactive character of a composition comprising the steps consisting of
(i) providing a perfume composition comprising perfume raw materials;
**characterized in that** the perfume raw materials comprises:
- from 0.01% to 50% by weight of a first group A of perfume raw materials having a LogP ≤ 2.5, and
- from 0.01% to 50% by weight of a second group B of perfume raw materials having a LogP ≥ 4
wherein the sum of perfume raw materials of group A and perfume raw materials of group B is greater than 35% by weight based on the total weight of the perfume raw materials.; and
(ii) subjecting said composition to wet conditions.

## Patentansprüche

1. Antiperspirant- oder Deodorantzusammensetzung, umfassend:
- einen Antiperspirant- oder Deodorantwirkstoff;
- gegebenenfalls einen Träger; und
- eine Parfümzusammensetzung, umfassend Parfümrohstoffe;
**dadurch gekennzeichnet, dass** die Parfümrohstoffe Folgendes umfassen:
- von 0,01-50 Gew.-% einer ersten Gruppe A von Parfümrohstoffen, die einen logP ≤ 2,5 aufweisen, und
- von 0,01-50 Gew.-% einer zweiten Gruppe B von Parfümrohstoffen, die einen logP > 4 aufweisen,
wobei die Summe der Parfümrohstoffe von Gruppe A und der Parfümrohstoffe von Gruppe B größer als 35 Gew.-% ist, basierend auf dem Gesamtgewicht der Parfümrohstoffe.

2. Zusammensetzung nach Anspruch 1, wobei die erste Gruppe von Parfümrohstoffen Parfümrohstoffe umfasst, die einen logP ≤ 2,5 aufweisen und die zweite Gruppe von Parfümverbindungen Parfümrohstoffe umfasst, die einen logP ≥ 4,5 aufweisen.

3. Zusammensetzung nach Anspruch 1 oder 2, wobei die Parfümrohstoffe zwischen 2-25 Gew.-% der ersten Gruppe A von Parfümrohstoffen und/oder zwischen 2-25 Gew.-% der zweiten Gruppe B von Parfümrohstoffen umfassen.

4. Zusammensetzung nach einem der vorhergehenden Ansprüche, wobei die Summe der Parfümrohstoffe von Gruppe A und der Parfümrohstoffe von Gruppe B, die einen Geruchsschwellenwert (GSW) ≤ 2 × 10⁻³ µg/l aufweisen, größer als 8 Gew.-% ist, basierend auf dem Gesamtgewicht der Parfümrohstoffe.

5. Zusammensetzung nach einem der Ansprüche 1-4, wobei die Parfümrohstoffe aus der ersten Gruppe A ausgewählt werden aus der Gruppe bestehend aus 2-(4-Methyl-1,3-thiazol-5-yl)-1-ethanol, 3-Ethoxy-4-hydroxybenzaldehyd, 2-Ethyl-3-hydroxy-4(4H)-pyranon, 4-(4-Hydroxyphenyl)-2-butanon, 7-Hydroxy-2-chromenon, Phenylethylalkohol, Anisaldehyd, Zimtalkohol, 4-Nonanolid, 2-Methoxy-4-(2-propen-1-yl)phenol, Methyl 2,4-dihydroxy-3,6-dimethylbenzoat, Phenylethylacetat, Vanillin, Isoeugenol, (3aRS,6SR,7aSR)-Perhydro-3,6-dimethyl-benzo[b]furan-2-on, Ethylbutyrat, Methylnaphtylketon, 7-Methyl-2H-1,5-benzodioxepin-3(4H)-on, 3-(1,3-Benzodioxol-5-yl)-2-methylpropanal, 2,4-Dimethyl-3-cyclohexen-1-carbaldehyd, (+-)-3-Phenylbutanal, Benzylaceton, 4-(3,4-Methylendioxyphenyl)-2-butanon, 5-Ethyl-4-hydroxy-2-methyl-furan-3-on, Ethylisobutyrat, Ethylmethylphenylglycidat, Ethyl 2-methyl-1,3-dioxolan-2-acetat, Benzolmethanol, alpha-Methylen, Acetat und Gemischen davon.

6. Zusammensetzung nach einem der Ansprüche 1-4, wobei die Parfümrohstoffe aus der zweiten Gruppe ausgewählt werden aus der Gruppe bestehend aus (4Z)-4-Dodecenal, 3,3-Dimethyl-5-(2,2,3-trimethyl-3-cyclopenten-1-yl)-4-penten-2-ol,{1-methyl-2-[(1,2,2-trimethylbicyclo[3.1.0]hex-3-yl)methyl]cyclopropyl}methanol, 2-Methylundecanal, (15,1'R)-2-[1-(3',3'-Dimethyl-1'-cyclohexyl)ethoxy]-2-methylpropylpropanoat, (3aR,5aS,9aS,9bR)-3a,6,6,9a-Tetramethyldodecahydronaphtho[2,1-b]furan, 1-(2,2,3,6-Tetramethyl-cyclohexyl)-3-hexanol, 1-(Octahydro-2,3,8,8-tetramethyl-2-naphtalenyl)-1-ethanon, (2e)-Tridec-2-ennitril, Verdylpropionat, Naphtho[2,1-b]furan, Dodecahydro3a,6,6,9a-tetramethyl, Patchoulol, Aldehyd C12, 1-[(1RS,6SR)-2,2,6-Trimethylcyclohexyl]-3-hexanol, (+)-(3R)-1-[(1R,6S)-2,2,6-Trimethylcyclohexyl]-3-hexanol, (3E,5Z)-1,3,5-Undecatrien, Pentadecenolid, (+-)-2-(4-Methyl-3-cyclohexen-1-yl)-2-propanthiol, Aldeyhyde Supra, ((-)-(2e)-2-Ethyl-4-[(1R)-2,2,3-trimethyl-3-cyclopenten-1-yl]-2-buten-1-ol, 2-Methyl-4-(2,2,3-trimethyl-3-cyclopenten-1-yl)-4-penten-1-ol, Methylionon, 3-Methyl-cyclopentadecanon, Hexylzimtaldehyd, (+-)-(1S,4aR,8S,8aR)-2,2,6,8-Tetramethyl-1,2,3,4,4a,5,8,8a-octahydro-1-naphthalenol, Oxacyclohexadecan-2-on, Muscenone Delta, Delta-Damascone und Gemischen davon.

7. Zusammensetzung nach einem der vorhergehenden Ansprüche, wobei es sich bei dem Träger um eine Flüssigkeit handelt und er ausgewählt ist aus der Gruppe bestehend aus Ethanol, Wasser, einem Tensid und Gemischen davon.

8. Zusammensetzung nach einem der Ansprüche 1 bis 6, wobei die Zusammensetzung wasserfrei ist.

9. Zusammensetzung nach einem der vorhergehenden Ansprüche, wobei die Parfümzusammensetzung zusätzlich zu den Parfümrohstoffen ein Lösungsmittel umfasst, ausgewählt aus der Gruppe bestehend aus Dipropylenglykol, Isopar M (Kohlenwasserstoffe C13-C14), Isopar L (Kohlenwasserstoffe C11-C13), Isopropylmyristat (Isopropyl-Tetradecanoat), Ethylcitrat (triethyl 2-hydroxy-1,2,3-propantricarboxylat), Triacetin (1,2,3-Propantriyltriacetat, 1,3-Propandiol, Gemisch aus Methyldihydroabietat und Methyltetrahydroabietat, pflanzlichen Ölen wie Mandelöl, Arganöl, Baumwollöl, Maisöl, Olivenöl, Sonnenblumenöl, Rizinusöl und Gemischen davon.

10. Zusammensetzung nach einem der vorhergehenden Ansprüche, wobei die Parfümzusammensetzung ferner zumindest 10 Gew.-% der Parfümrohstoffe von Gruppe C umfasst, die einen log P, der zwischen 2,5 und 4 liegt und/oder einen Geruchsschwellenwert (GSW) von ≤ 2 × 10⁻³ µg/l aufweisen, vorzugsweise ausgewählt aus der Gruppe bestehend aus Linalylacetat, Benzylbenzoat, Dihydromyrcenol, Linalol, Sclareolat, Ethylacetoacetat und Gemischen davon.

11. Zusammensetzung nach einem der vorhergehenden Ansprüche, wobei das Antiperspirant oder Deodorant ausgewählt wird aus der Gruppe bestehend aus einer Körpersprayformulierung, einer festen Formulierung, einer Roll-on-Formulierung und einer AerosolFormulierung.

12. Parfümzusammensetzung, umfassend Parfümrohstoffe; **dadurch gekennzeichnet, dass** die Parfümrohstoffe Folgendes umfassen:
- von 0,01-50 Gew.-% einer ersten Gruppe A von Parfümrohstoffen, die einen logP ≤ 2,5 aufweisen, und
- von 0,01-50 Gew.-% einer zweiten Gruppe B von Parfümrohstoffen, die einen logP > 4 aufweisen,
wobei die Summe der Parfümrohstoffe von Gruppe A und der Parfümrohstoffe von Gruppe B größer als 35 Gew.-% ist, basierend auf dem Gesamtgewicht der Parfümrohstoffe.

13. Verwendung einer Zusammensetzung, umfassend Parfümrohstoffe;
**dadurch gekennzeichnet, dass** die Parfümrohstoffe Folgendes umfassen:
- von 0,01-50 Gew.-% einer ersten Gruppe A von Parfümrohstoffen, die einen logP ≤ 2,5 aufweisen, und
- von 0,01-50 Gew.-% einer zweiten Gruppe B von Parfümrohstoffen, die einen logP > 4 aufweisen,
wobei die Summe der Parfümrohstoffe von Gruppe A und der Parfümrohstoffe von Gruppe B größer als 35 Gew.-% ist, basierend auf dem Gesamtgewicht der Parfümrohstoffe, um den olfaktorischen Charakter der Zusammensetzung unter nassen Bedingungen zu modifizieren.

14. Verfahren zum Modifizieren des olfaktorischen Charakters einer Zusammensetzung, umfassend die folgenden Schritte, bestehend aus:
(i) Bereitstellen einer Parfümzusammensetzung, umfassend Parfümrohstoffe;
**dadurch gekennzeichnet, dass** die Parfümrohstoffe Folgendes umfassen:
- von 0,01-50 Gew.-% einer ersten Gruppe A von Parfümrohstoffen, die einen logP ≤ 2,5 aufweisen, und
- von 0,01-50 Gew.-% einer zweiten Gruppe B von Parfümrohstoffen, die einen logP > 4 aufweisen wobei die Summe der Parfümrohstoffe von Gruppe A und der Parfümrohstoffe von Gruppe B größer als 35 Gew.-% ist, basierend auf dem Gesamtgewicht der Parfümrohstoffe, und
(ii) Aussetzen der Zusammensetzung nassen Bedingungen.

## Revendications

1. Composition d'antitranspirant ou de déodorant comprenant :
- une matière active antitranspirante ou déodorante ;
- éventuellement un support ; et
- une composition de parfum comprenant des matières premières de parfum ;
**caractérisée en ce que** les matières premières de parfum comprennent
- de 0,01 % à 50 % en poids d'un premier groupe A de matières premières de parfum ayant un LogP ≤ 2,5, et
- de 0,01 % à 50 % en poids d'un deuxième groupe B de matières premières de parfum ayant un LogP ≥ 4,
la somme des matières premières de parfum du groupe A et des matières premières de parfum du groupe B étant supérieure à 35 % en poids sur la base du poids total des matières premières de parfum.

2. Composition selon la revendication 1, le premier groupe de matières premières de parfum comprenant une matière première de parfum ayant un LogP ≤ 2,5 et le deuxième groupe de composés parfumants comprenant des matières premières de parfum ayant un LogP ≥ 4,5.

3. Composition selon la revendication 1 ou 2, les matières premières de parfum comprenant entre 2 et 25 % en poids du premier groupe A de matières premières de parfum et/ou entre 2 et 25 % en poids du deuxième groupe B de matières premières de parfum.

4. Composition selon l'une quelconque des revendications précédentes, la somme des matières premières de parfum du groupe A et des matières premières de parfum du groupe B ayant un seuil de détection d'odeur (ODT) ≤ 2 × 10⁻³ µg/L étant supérieure à 8 % en poids sur la base du poids total des matières premières de parfum.

5. Composition selon l'une quelconque des revendications 1 à 4, les matières premières de parfum du premier groupe A étant choisies dans le groupe constitué par 2-(4-méthyl-1,3-thiazol-5-yl)-1-éthanol, 3-éthoxy-4-hydroxybenzaldéhyde, 2-éthyl-3-hydroxy-4(4H)-pyranone, 4-(4-hydroxyphényl)-2-butanone, 7-hydroxy-2-chroménone, alcool phényléthylique, aldéhyde anisique, alcool cinnamique, 4-nonanolide, 2-méthoxy-4-(2-propén-1-yl)phénol, 2,4-dihydroxy-3,6-diméthylbenzoate de méthyle, acétate de phényléthyle, vanilline, isoeugénol, (3aRS,6SR,7aSR)-perhydro-3,6-diméthyl-benzo[b]furan-2-one, butyrate d'éthyle, méthylnaphtylcétone, 7-méthyl-2H-1,5-benzodioxépine-3(4H)-one, 3-(1,3-benzodioxol-5-yl)-2-méthylpropanal, 2,4-diméthyl-3-cyclohexène-1-carbaldéhyde, (+-)-3-phénylbutanal, benzylacétone, 4-(3,4-méthylènedioxyphényl)-2-butanone, 5-éthyl-4-hydroxy-2-méthyl-furan-3-one, isobutyrate d'éthyle, méthylphénylglycidate d'éthyle, 2-méthyl-1,3-dioxolane-2-acétate d'éthyle, benzèneméthanol, alpha-méthylène, acétate, et des mélanges correspondants.

6. Composition selon l'une quelconque des revendications 1 à 4, les matières premières de parfum du deuxième groupe étant choisies dans le groupe constitué par (4Z)-4-dodécénal, 3,3-diméthyl-5-(2,2,3-triméthyl-3-cyclopentén-1-yl)-4-pentén-2-ol, {1-méthyl-2-[(1,2,2-triméthylbicyclo[3.1.0]hex-3-yl)méthyl]cyclopropyl}méthanol, 2-méthylundécanal, propanoate de (1S,1'R)-2-[1-(3',3'-diméthyl-1'-cyclohexyl)éthoxy]-2-méthylpropyle, (3aR, 5aS, 9aS, 9bR) -3a, 6, 6, 9a-tétraméthyldodécahydronaphto[2,1-b]furane, 1-(2,2,3,6-tétraméthyl-cyclohexyl)-3-hexanol, 1-(octahydro-2,3,8,8-tétraméthyl-2-naphtalényl)-1-éthanone, (2e)-tridéc-2-ènenitrile, propionate de verdyle, naphto[2,1-b]furane, dodécahydro3a,6,6,9a-tétraméthyle, patchoulol, aldéhyde C12, 1-[(1RS,6SR)-2,2,6-triméthylcyclohexyl]-3-hexanol, (+)-(3R)-1-[(1R,6S)-2,2,6-triméthylcyclohexyl]-3-hexanol, (3E,5Z)-1,3,5-undécatriène, pentadécénolide, (+-)-2-(4-méthyl-3-cyclohexén-1-yl)-2-propanethiol, aldéyhyde supra, ((-)-(2e)-2-éthyl-4-[(1R)-2,2,3-triméthyl-3-cyclopentén-1-yl]-2-butén-1-ol, 2-méthyl-4-(2,2,3-triméthyl-3-cyclopentén-1-yl)-4-pentén-1-ol, méthyl-ionone, 3-méthyl-cyclopentadécanone, aldéhyde hexylcinnamique, (+-)-(1S,4aR,8S,8aR)-2,2,6,8-tétraméthyl-1,2,3,4,4a,5,8,8a-octahydro-1-naphtalénol, oxacyclohexadécan-2-one, muscénone delta, delta damascone et des mélanges correspondants.

7. Composition selon l'une quelconque des revendications précédentes, le support étant liquide et choisi dans le groupe constitué par l'éthanol, l'eau, un tensioactif et des mélanges correspondants.

8. Composition selon l'une quelconque des revendications 1 à 6, la composition étant anhydre.

9. Composition selon l'une quelconque des revendications précédentes, la composition de parfum comprenant en plus des matières premières de parfum un solvant choisi dans le groupe constitué par le dipropylèneglycol, l'Isopar M (hydrocarbures en C13-C14), l'Isopar L (hydrocarbures en C11-C13), le myristate d'isopropyle (tétradécanoate d'isopropyle), le citrate d'éthyle (2-hydroxy-1,2,3-propanetricarboxylate de triéthyle), la triacétine (triacétate de 1,2,3-propanetriyle), le 1,3-propanediol, un mélange de dihydroabiétate de méthyle et de tétrahydroabiétate de méthyle, des huiles végétales telles que l'huile d'amande, l'huile d'argan, l'huile de coton, l'huile de maïs, l'huile d'olive, l'huile de tournesol, l'huile de ricin et des mélanges correspondants.

10. Composition selon l'une quelconque des revendications précédentes, la composition de parfum comprenant en outre au moins 10 % en poids de matières premières de parfum du groupe C ayant un logP compris entre 2,5 et 4 et/ou un seuil de détection d'odeur (ODT) de ≤ 2 × 10⁻³ pg/L, préférablement choisies dans le groupe constitué par l'acétate de linalyle, le benzoate de benzyle, le dihydromyrcénol, le linalol, le sclaréolate, l'acétoacétate d'éthyle et des mélanges correspondants.

11. Composition selon l'une quelconque des revendications précédentes, l'antitranspirant ou le déodorant étant choisi dans le groupe constitué par une formulation de spray corporel, une formulation solide, une formulation pour système à bille et une formulation d'aérosol.

12. Composition de parfum comprenant des matières premières de parfum ;
**caractérisée en ce que** les matières premières de parfum comprennent :
- de 0,01 % à 50 % en poids d'un premier groupe A de matières premières de parfum ayant un LogP ≤ 2,5, et
- de 0,01 % à 50 % en poids d'un deuxième groupe B de matières premières de parfum ayant un LogP ≥ 4,
la somme des matières premières de parfum du groupe A et des matières premières de parfum du groupe B étant supérieure à 35 % en poids sur la base du poids total des matières premières de parfum.

13. Utilisation d'une composition comprenant des matières premières de parfum ;
**caractérisée en ce que** les matières premières de parfum comprennent :
- de 0,01 % à 50 % en poids d'un premier groupe A de matières premières de parfum ayant un LogP ≤ 2,5, et
- de 0,01 % à 50 % en poids d'un deuxième groupe B de matières premières de parfum ayant un LogP ≥ 4
la somme des matières premières de parfum du groupe A et des matières premières de parfum du groupe B étant supérieure à 35 % en poids sur la base du poids total des matières premières de parfum pour modifier le caractère olfactif de ladite composition dans des conditions humides.

14. Procédé pour la modification du caractère olfactif d'une composition comprenant les étapes consistant à :
(i) fournir une composition de parfum comprenant des matières premières de parfum ;
**caractérisé en ce que** les matières premières de parfum comprennent :
- de 0,01 % à 50 % en poids d'un premier groupe A de matières premières de parfum ayant un LogP ≤ 2,5, et
- de 0,01 % à 50 % en poids d'un deuxième groupe B de matières premières de parfum ayant un LogP ≥ 4
la somme des matières premières de parfum du groupe A et des matières premières de parfum du groupe B étant supérieure à 35 % en poids sur la base du poids total des matières premières de parfum ; et
(ii) soumettre ladite composition à des conditions humides.
